# EUROPÄISCHE PATENTANMELDUNG

(11) **EP 1 505 179 A1**
(43) Veröffentlichungstag der Anmeldung: **09.02.2005**
(21) Anmeldenummer: 04001722.0
(22) Anmeldetag: 27.01.2004
(51) Int. Cl.: C30B 7/00, B01J 19/00, G01N 33/68

(54) **VERFAHREN ZUR IDENTIFIZIERUNG VON SCHWACH BINDENDEN MOLEKÜLFRAGMENTEN MIT LIGANDENEIGENSCHAFTEN, WOBEI DIE MOLEKÜLFRAGMENTE ALS MIKROTROPFEN EINER ENTSPRECHENDEN LÖSUNG AUF DEN KRISTALL AUFGEBRACHT WERDEN**

(30) Priorität: 06.08.2003 DE 10336110
(71) Anmelder: Proteros Biostructures GmbH, 82152 Martinsried (DE)
(72) Erfinder: Neuefeind, Thorsten, Dr., 82131 Gauting (DE); Brandstetter, Hans, Dr., 82131 Gauting (DE)
(74) Vertreter: Graf von Stosch, Andreas, Dr. rer. nat.

(57) **Zusammenfassung**

Die vorliegende Erfindung betrifft ein Verfahren zum Behandeln eines Kristalls mit einer Lösung, wobei die Lösung eine oder mehr Molekülspezies enthält und wobei die Moleküle der Molekülspezies typischerweise ein Molekulargewicht von < 500 Da aufweisen. Mit Hilfe diese Verfahrens können auf Targetstrukturen schwach bindende kleine Moleküle oder Molekülfragmente identifiziert und deren Bindungsposition durch nachfolgende röntgenkristallographische Untersuchungen ermittelt werden.

## Beschreibung

Die vorliegende Erfindung betrifft ein Verfahren zum Behandeln eines Kristalls mit einer Lösung, wobei die Lösung eine oder mehr Molekülspezies enthält und wobei die Moleküle der Molekülspezies typischerweise ein Molekulargewicht von < 500 Da aufweisen. Mit Hilfe diese Verfahrens können auf Targetstrukturen schwach bindende kleine Moleküle oder Molekülfragmente identifiziert und deren Bindungsposition durch nachfolgende röntgenkristallographische Untersuchungen ermittelt werden.

Im Stand der Technik ist eine Vielzahl von Verfahren beschrieben, um mit Hilfe von in vitro Testverfahren oder auch mit Hilfe von Methoden zur Strukturaufklärung, beispielsweise über röntgenkristallographische Experimente oder NMR- Experimente, Liganden makromolekularer physiologischer Substanzen zu identifizieren und/oder zu charakterisieren. Derartige Target-Strukturen können bspw. insbesondere Proteine, die im Stoffwechsel, bei der intra- oder extrazellulären Signalweitergabe, beispielsweise als membranständige Rezeptoren, physiologisch Bedeutung haben, sein. Herkömmliche Verfahren zur Identifizierung derartiger Liganden setzen Substanzbibliotheken voraus, so dass eine große Vielzahl an Substanzen, die potentiell als Liganden für derartige Target-Strukturen in Betracht kommen, im Rahmen von entsprechenden Messungen eingesetzt werden müssen. Obwohl derartige Testmethoden im Hochdurchsatzverfahren ("High-Throughput-Screening", HTS) durchgeführt werden, haben die Resultate derartiger Hochdurchsatzversuche, unabhängig davon, ob sie durch In-vitro-Testverfahren oder ggf. auf strukturbiologischem Weg erfolgen, bislang nicht zu durchgreifenden Resultaten geführt. Neben einer Anzahl von weiteren Gründen mag für die vergleichsweise geringe Ausbeute an auf diese Weise im Hochdurchsatzverfahren identifizierten, neuen Leitsubstanzen für die Arzneimittelforschung, also der Identifizierung neuer Liganden, ausschlaggebend sein, dass nur auf Grund kleiner sterischer oder geometrischer Inkompatibilitäten der Testsubstanzen mit der Target-Struktur, z.B. im Bereich einer Bindungstasche oder auch im Eintrittsbereich ("entry site") in das Innere einer Struktur, bereits ein positives Signal (beispielsweise ein Signal, das die Bindung der Testsubstanzen an die Target-Struktur indiziert) unterbleibt, obwohl bereits geringfügigste strukturelle Modifizierungen der Testsubstanzen zu einem Bindungssignal geführt hätten.

Um derartige experimentelle Fehlschläge zu vermeiden, ist im Stand der Technik das Konzept entwickelt worden, Hochdurchsatz-Testverfahren mit Testsubstanzen, deren sterische Flexibilität erhöht ist, durchzuführen. Eine Möglichkeit, eine größere Trefferquote bei Hochdurchsatzverfahren zur Ermittlungen von bindenden Leitsubstanzen zu erreichen, besteht darin, Teilstrukturen chemischer Verbindungen (sog. "Fragmente" klassischer bspw. organisch-chemischer Moleküle) als Testsubstanzen einzusetzen ("fragmentorientierter Ansatz"). Im Anschluß werden einzelne an die Target-Struktur in Nachbarschaft gebundene Fragmente über Linkerelemente chemisch miteinander kombiniert und damit durch Kombination kleiner oder kleinster Fragmente bausteinartig Leitsubstanzen mit Liganden-Eigenschaft zusammengesetzt.

Um einerseits solche an die Target-Struktur gebundenen Fragmente zu identifizieren, die in räumlicher Nähe zueinander liegen und um andererseits in stereo-chemisch sinnvoller Weise Linker-Strukturen zwischen den einzelnen gebundenen Fragmenten konstruieren zu können, die im Einklang mit den Strukturvorgaben des Targets stehen, ist es für den fragmentbasierten Ansatz jedoch erforderlich, Strukturinformationen zum Bindungsplatz mindestens eines, vorzugsweise mehrerer Fragmente auf der Target-Struktur zu gewinnen.

Hierbei sind im Stand der Technik Computer-basierte Verfahren beschrieben, mit deren Hilfe Fragmente zu neuen potentiellen Liganden einer Target-Struktur zusammengesetzt werden können. Ein derartiger computerexperimenteller Ansatz wird bspw. von Wang et al. (Journal of Molecular Modeling, 2000, 6, 498) offenbart, wobei kleine Strukturabschnitte, also die Fragmente, zunächst ausgewählt und dann schrittweise in bspw. der Bindungstasche der Target-Struktur kombiniert werden. Zahlreiche Programme oder Programmpakete, die ein derartiges - in der Literatur als "de novo-Design" beschriebenes Vorgehen ermöglichen, sind im Stand der Technik verfügbar, beispielsweise die Programme GROW (Moon et al. , Proteins, 1991, II, 314), LUDI (Bohm, J. Comp.-aided Molecul. Des., 1992, 6, 61), LEAPFROG (Cramer, integrated in the SYBYL program package, 1996, Tripos, St. Louis, MO, USA), PROLIGAND (Clark et al, J. Comp.-aided Molecul. Des., 1995, 9, 13 oder 213; Clark et al. , J. Chem. Inf. Comput. Sci., 1995, 35, 914). Derartige computerexperimentelle Ansätze haben jedoch grundsätzlich den Nachteil, dass computerexperimentelle Ergebnisse nur bedingt die realen Verhältnisse abbilden und stets laborexperimentell überprüft werden müssen. Auch kleine Abweichungen bei der Simulation der zwischen den Fragmenten und der Targetstruktur bestehenden Interaktionen durch die im Computerexperiment getroffenen theoretischen Annahmen, bspw. bei den eingesetzten Kraftfeldern, können zu in der Praxis völlig irrelevanten Ergebnissen bei der Computer-experimentellen Leitsubstanzidentifizierung führen.

Strukturinformationen der erforderlichen Art zur Durchführung eines fragmentbasierten Ansatzes können allerdings auch durch reale Experimente mit der Target-Struktur mit Hilfe biophysikalischer Verfahren, beispielsweise mit Hilfe der Röntgenkristallographie (Blundell, 2002) erhalten werden. Im Stand der Technik sind Verfahren vorbeschrieben, die einen fragmentbasierten Ansatz in Kombination mit röntgenkristallographischen Experimenten beschreiben. So etwa wird von Nienaber (Nature Biotechnology vol. 18, 2000, 1105 ff.) ein kristallographisches "Screening"-Verfahren offenbart, das auf einer "Soaking"-Technik beruht, d.h. bei welchem die Target-Struktur, insbesondere also ein Target-Protein, in kristallisierter Form vorliegt und der Proteinkristall einer Lösung ausgesetzt wird, die verschiedene Fragmente, d.h. chemische Teilstrukturen, wie z.B. substituierte Phenylringe oder substituierte bizyklische Ringsysteme, enthält. Die jeweilige Elektronendichte der einzelnen Komponenten in der Lösung ist bekannt, so dass spezifische Elektronendichten, die nach röntgenkristallographischen Experimenten mit dem "gesoakten" Kristall erhalten werden, eindeutig einzelnen in der "Soaking"-Lösung enthaltenen Fragmenten zugeordnet werden können. Durch einen Vergleich der Elektronendichtekarten vor dem "Soaking" des Kristalls und nach dem "Soaking" des Kristalls wird es möglich, in einer Differenzelektronendichtekarte Bindungsplätze der Fragmente auf der Target-Struktur zu identifizieren. Schließlich können dann nach Maßgabe stereochemischer Vorgaben die einzelnen Fragmente zu einem potentiellen Liganden zusammengesetzt werden. Diese von Nienaber et al. entwickelte Form des "Screenings" von chemischen Fragmenten zur Entwicklung von neuen Liganden ist jedoch in seiner Anwendung beschränkt. Nicht nur ist bei einem derartigen Verfahren ein "Soaking"-Schritt dem röntgenkristallographischen Experiment vorgeschaltet, welches Schwierigkeiten aufwerfen kann. Bspw. kann nicht nur die für das nachfolgende röntgenkristallographische Experiment erforderliche Unversehrtheit des Kristalls durch das "Soaking" beeinträchtigt werden, sondern der beschriebene Ansatz kann auch nicht den Anforderungen eines Hochdurchsatzexperiments gerecht werden. Insbesondere müssen die einzelnen in der "Soaking"-Lösung enthaltenen Fragmente aufgrund ihrer jeweiligen Elektronendichte unterscheidbar sein, so dass die ohnehin der Zahl nach beschränkten Fragmente in einer "Soaking"-Lösung auch spezifisch ausgewählt werden müssen.

Ein weiterer Nachteil der vorbeschriebenen Vorgehensweise ist jedoch auch die Tatsache, dass einem solchen Verfahren auch die geringe Bindungsaffinität der kleinen Fragmente an die Target-Struktur entgegensteht. Dieser Nachteil liegt indes auch einer weiteren alternativen Methode, die Bindung von Fragmenten an z.B. Protein-Targets röntgenkristallographisch zu untersuchen, zugrunde. Bei dieser Alternative werde die Fragmente bereits mit einem Target, z.B. Protein, zum Erhalt eines Cokristalls kristallisiert. Darüber hinaus erweist sich ein solches Verfahren für den Hochdurchsatz als ungeeignet, da eine Co-Kristallisation mit jedem einzelnen Fragment erfolgen muss, so dass eine Vielzahl von Co-Kristallen erzeugt werden muß, um die Fragmentbindungsplätze röntgenkristallographisch bestimmen zu können.

Von Lesuisse et al. (J. Med. Chem. 2002, 45, 2379) wird ein alternatives Verfahren offenbart, mit dem gleichfalls experimentell ein fragmentorientierter Ansatz verfolgt wird, wobei das Verfahren allerdings seiner Struktur nach die Verfeinerung von Wirkstoffen zum Ziel hat und nicht der Wirkstoffidentifizierung als solcher im engeren Sinn dient.

Obwohl also röntgenkristallographische Verfahren inzwischen in vielen Bereichen als Screening-Methode eingesetzt werden können, sind bislang keine Verfahren verfügbar, die mit hoher Ausbeute die Identifizierung von neuen Liganden fragmentbasiert erlauben könnten.

Es ist daher die Aufgabe der vorliegenden Erfindung, ein Verfahren bereitzustellen, das mit Hilfe röntgenkristallographischer Methoden dazu geeignet ist, an die Target-Struktur nur schwach bindende Fragmente zu identifizieren und auf diesem Weg die Identifizierung neuer Liganden, die als Arzneimittelwirkstoffe in Betracht kommen könnten, in - verglichen mit dem Stand der Technik großer Ausbeute - erlaubt.

Diese Aufgabe wird durch ein erfindungsgemäßes Verfahren nach Anspruch 1 gelöst. Vorteilhafte Ausgestaltung dieses erfindungsgemäßen Verfahrens sind in den Unteransprüchen enthalten.

Die Aufgabe wird durch eine Verfahren zum Behandeln eines Kristalls mit einer Lösung, enthaltend eine oder mehr Molekülspezies, wobei die Moleküle der einen oder mehr Molekülspezies ein Molekulargewicht von < 500 Da aufweisen, mit den folgenden Schritten gelöst (a) es wird der Kristall auf einer Halterungsvorrichtung befestigt und danach werden (b) Mikrotropfen der Flüssigkeit auf den Kristall aufgebracht. Eine vorteilhafte, verfahrensgemäß einsetzbare Halterungsvorrichtung ist in der Anmeldung offenbart.

Die auf den Kristall aufgebrachten Lösungen enthalten also typischerweise kleine Moleküle oder Molekülfragmente, beispielsweise substituierte Ringverbindungen, aromatisch oder nicht-aromatisch, ggf. auch als Heterozyklen (bspw. Imidazol, Thiazol, Purin, Pyrimidin, Pyridyl etc), oder aus der organischen Chemie bekannte kleine lineare Fragmente, die ggf. auch typische funktionelle Gruppen aufweisen können, beispielsweise eine oder mehr Amino- oder Carboxyl- oder Carbonyl- oder Aldehyd- oder Nitro- oder Hydroxyfunktionen, ggf. auch hydrophobe Alkylgruppen (verzweigt oder unverzweigt). In der aufgetragenen Lösung kann eine derartige Molekülspezies enthalten sein, also nur beispielsweise eine Spezies eines spezifischen substituierten Phenylrings oder aber es können auch zwei oder mehr oder derartige Spezies in der Lösung auftreten. Die in der Lösung enthaltenen Moleküle oder Molekülfragmente werden vorteilhafterweise ein Molekulargewicht < 200 Da aufweisen, weiter bevorzugt von <100 Da. Schließlich sind Fragmente bevorzugt, die eine Bindungsaffinität an den Zielstruktur zwischen 10⁻³ und 10⁻⁵ M aufweisen, besonders bevorzugt zwischen 10⁻³ und 10⁻⁴ M. Die Fragmente weisen vorteilhafterweise eine Struktur auf, die es erlaubt, Wechselwirkungen mit der Zielstruktur auszubilden, bspw. eine hydrophobe Wechselwirkung, eine Wasserstoffbrücke und/oder eine aromatische-aromatische Wechselwirkung. Besonders bevorzugt sind solche Fragmente, die mindestens eine funktionelle Gruppe, wie vorstehend genannt, aufweisen. Weiterhin werden Fragmente, die in der auf den Kristall aufzubringenden Lösung eingesetzt werden, typischerweise nicht mehr als 3 frei rotierbare Bindungen aufweisen, ganz besonders typisch 2 oder 3 frei rotierbare Bindungen.

Bei dem Kristall, der mit der Lösung behandelt wird, wird es sich um einen Kristall mit einer Targetstruktur handeln, typischerweise um einen Proteinkristall, wobei der Kristall die kristallisierten Targetstrukturen in jeder denkbaren Raumgruppe enthalten kann. Beispielsweise können die kristallisierten Strukturen in einer hexagonalen, kubischen, monoklinen, tetragonalen, triklin oder trigonalen auftreten.

Der Kristall kann grundsätzlich auf jeder beliebigen Halterungsvorrichtung aufgebracht werden, besonders bevorzugt sind jedoch solche Halterungsvorrichtungen, wie sie etwa in der deutschen Patentschrift DE 198 42 797 C1 beschrieben sind, welche Bestandteil der vorliegenden Offenbarung ist. Hierbei wird der Kristall in der Art des "Free Mounting Systems" auf der Halterungsvorrichtung befestigt. Um jegliche Veränderungen des Kristalls während seiner Verweilzeit auf der Halterungsvorrichtung ausschließen zu können, wird der Kristall vorteilhafter Weise bei einem erfindungsgemäßen Verfahren in einer definierten Umgebung gehalten, die beispielsweise die entsprechende definierte Feuchte, die der Kristall benötigt, um seine Struktur während des Behandlungs- bzw. Messvorgangs zu wahren, bereitstellt. Die entsprechende konstant bleibende Umgebung des Kristalls kann beispielsweise mit Hilfe eine Gasstroms definierter Zusammensetzung erzeugt werden, wobei vorzugsweise der Gasstrom aus einem Luftstrom mit kontrollierter Luftfeuchtigkeit besteht. Derartige Verfahren zur Sicherstellung einer gleichbleibenden Umgebung des Kristalls sind in der DE 102 32 172.8 beschrieben und werden insoweit vollinhaltlich in die vorliegende Offenbarung einbezogen.

Der Kristall wird nach dem erfindungsgemäßen Verfahren mit Mikrotropfen behandelt, wobei die Behandlung vorzugsweise mit einer Vorrichtung, die ein "Mikrodosiersystem" aufweist, erfolgt, wie sie nachfolgend in der vorliegenden Anmeldung beschrieben wird.

Das Aufbringen der Lösung auf den Kristall kann ohne eine entsprechende definierte Umgebung erfolgen oder es kann gleichzeitig mit dem Auftropfen, wie oben beschrieben, vorzugsweise eine gleichmäßige Umgebung mit Hilfe eines beispielsweise Gasstroms, hergestellt werden. Wird die kleine Moleküle oder Molekülfragmente enthaltende Lösung gleichzeitig mit dem Gasstrom aufgetropft, ist eine Abstimmung zwischen der Zufuhr des beispielsweise Gasstroms und der Art des Auftropfvorgangs bevorzugt. Hierbei wird typischerweise ein aufeinander abgestimmter Regelmechanismus eingesetzt werden, um Flüssigkeits- bzw. Volumensveränderungen oder andere störende Einflüsse zu vermeiden. Ganz besonders bevorzugt ist es dann, wenn etwa die Luftfeuchtigkeit des Gasstroms und die Frequenz, mit der die Tropfen durch das Mikrodosiersystem auf den Kristall aufgetropft werden, während des Auftropfens so aufeinander abgestimmt werden, daß der Kristall möglichst wenig belastet wird. Der beispielsweise zur Aufrechterhaltung einer gleichbleibenden Atmosphäre um den Kristall herum verwendete Gasstrom kann mindestens eine weitere funktionale Komponente enthalten, beispielsweise einen Lösungsvermittler, der die auf den Kristall aufzubringende Substanz in Lösung hält und damit das Eindringen der Substanz in den Kristall verbessert. Auch andere Komponenten, die bspw. die Ausfällung der Fragmente auf dem Kristall vermeiden, werden bevorzugt zugeführt.

Die auf den Kristall aufgetragenen Mikrotropfen sind typischerweise kleiner als das Volumen des behandelten Kristalls, vorzugsweise weist ein solcher Mikrotropfen ein Volumen zwischen 1 nl und 100 pl, bevorzugt zwischen 100 pl und 20 pl und noch stärker bevorzugt zwischen 20 pl und 4 pl auf. Derartige Tropfengrößen werden durch ein Mikrodosiersystem auf den Kristall aufgebracht, wie es nachfolgend in der vorliegenden Anmeldung als Vorrichtung beschrieben wird und im Rahmen des erfindungsgemäßen Verfahrens eingesetzt werden kann.

Typischerweise wird ein erfindungsgemäßes Verfahren durchgeführt, indem die die Molekülspezies enthaltende und auf den Kristall aufgebrachte Lösung eine wässrige Lösung oder zumindest eine teilweise ein organisches Lösungsmittel umfassende Lösung ist. Derartige organische Lösungsmittel können, sofern mit der wässrigen Lösung mischbar, einen Volumensanteil von mindestens 5-% der aufgebrachten Lösung ausmachen, vorzugsweise zwischen 5 und 95 Vol.-% der aufgebrachten Lösung. Bevorzugt wird der Anteil des organischen Lösungsmittels zwischen 20 und 80 Vol.-% liegen, ganz besonders bevorzugt zwischen 30 und 70 Vol.-%. Grundsätzlich können also als aufzubringende Lösung Mischungen aus Wasser und mindestens einem organischen Lösungsmittel eingesetzt werden.

Die Lösung, die die mindestens eine Molekülspezies enthält, kann aber auch ein organisches Lösungsmittel ohne Beimengungen von Wasser sein. Ganz besonders bevorzugt sind solche Lösungen, die ein weitgehend vollständig flüchtiges Lösungsmittel umfassen, wodurch ein Akkumulieren der einen oder mehr aufgebrachten Molekülspezies auf dem Kristall möglich wird, wenn die Auftragungskinetik der aufgetragenen Lösung entsprechend an die Abdampfkinetik des flüchtigen Lösungsmittels angepaßt wird. Insbesondere vorteilhaft ist das erfindungsgemäße Verfahren im Hinblick auf die zu erreichenden Konzentrationen der Moleküle oder Molekülfragmente, wobei die Konzentration einer Molekülspezies im entsprechend vorzugsweise frei montierten Kristall in einem Bereich von ca. 10⁻¹ bis 10⁻³ M in einer vorteilhaften Ausführungsform liegt. Typischerweise liegt die akkumulierte Konzentration der Moleküle oder Molekülfragmente in einem Bereich von ca. 7 x 10⁻¹ bis 3 x 10⁻² M.

Organische Lösungsmittel als Trägerflüssigkeit der aufzubringenden Lösung zu verwenden, ist auch deshalb besonders vorteilhaft, weil die aufzubringenden Moleküle oder Molekülfragmente aufgrund ihrer hydrophoben oder partiell hydrophoben Eigenschaften in wässriger Lösung nur schwer löslich sind und daher in einer wässrigen Lösung typischerweise nicht auf den Kristall aufgetropft werden können. Besonders vorteilhafte Lösungsmittel als Trägerflüssigkeit der aufzubringenden Lösung sind solche organischen Lösungsmittel, die bei einer Temperatur von weniger als 100°C sieden und (vorzugsweise zu mindestens 70%, noch stärker bevorzugt zu mindestens 80%, am stärksten bevorzugt zu mindestens 90%) flüchtig sind. Im Falle des Einsatzes von nicht-wässrigen Lösungsmitteln kann es sich um Mischungen von mindestens zwei organischen Lösungsmitteln handeln oder um reine organische Lösungen. Die Lösungsmittel können bspw. ausgewählt sein aus einer Gruppe, bestehend aus DMSO, Trifluorethanol, Aceton, Chloroform, Ethanol und/oder Methanol.

Wie oben erwähnt, wird vorzugsweise, um einen Kristall während seiner Verweildauer auf der Halterungsvorrichtung in einer konstanten Umgebung zu halten, ein dynamisches Gleichgewicht zwischen Zuspeisung von Lösungsmittel und einem Feuchtestrom einerseits und dem Abdampfen von Kristallflüssigkeit bzw. dem als Trägerflüssigkeit dienenden Lösungmittel der aufgetragenen Lösung andererseits angestrebt. Bevorzugt ist es hierbei, wenn das Abdampfen des Kristallwassers, das zu einem Austrocknen des Kristalls führen kann, durch einen zumindest teilweise wasserhaltigen Feuchtestrom kompensiert werden kann. Alternativ oder ggf. zusätzlich kann das Kristallwasser auch durch Aufbringen von Flüssigkeit eines Mikrodosiersystems der nachfolgend beschriebenen Art ausgeglichen werden. Hierbei kann es sich um ein Mikrodosiersystem handeln, mit dem auch die Moleküle oder Molekülfragmente in Lösung aufgebracht werden oder auch um mindestens ein weiteres Mikrodosiersystem, dessen Aufgabe nicht das Aufbringen von Molekülspezies, sondern ausschließlich die (besonders bevorzugte wässrige) Lösungsmittelzuspeisung ist.

Um das dynamische Gleichgewicht herzustellen, sollte der Kristall während des Behandlungsvorgangs beobachtet werden, insbesondere sollte gegebenenfalls eine Volumenzunahme des Kristalls ermittelt werden. Diese Volumenzunahme könnte darauf beruhen, dass das die Moleküle enthaltende Lösungsmittel(gemisch) in erhöhtem Maße in den Kristall hineindiffundiert, wobei osmotische Effekte des Feuchtestroms gleichfalls eine Volumenzunahme bewirken können. In einer bevorzugten Ausführungsform wird während der Behandlung des Kristalls die Flächenprojektion desselben zur Beobachtung der Volumensentwicklung mitverfolgt. Idealerweise beträgt die Zunahme der Flächenprojektion des Kristalls weniger als 20 %, noch stärker bevorzugt weniger als 10 %. Gleichwohl kann auch, ohne dass eine kritische Zunahme des Kristallvolumens im Wege der Flächenprojektion beobachtet wird, die mikroskopische Ordnung des Kristalls durch das Austropfen der Lösung gefährdet werden. Bevorzugt im Rahmen des vorliegenden Verfahrens werden daher Röntgendiffraktionsexperimente durchgeführt, wobei das Beugungsbild eine Aussage über die mikroskopische Ordnung zulässt. Besonders bevorzugt werden die Röntgendiffraktionsexperimente in einer zeitlichen Abfolge regelmäßig durchgeführt, so dass dieser Parameter während des Auftragens unter Beobachtung bleibt.

Zur Herstellung des dynamischen Gleichgewichts müssen weiterhin neben dem Tropfenvolumen auch die Menge der aufgetragenen Lösung (welche wiederum von der Konzentration der in der Lösung enthaltenen Molekülspezies abhängt, wobei schließlich wiederum die erforderliche Konzentration der Molekülspezies von der Konzentration der Proteinbindungsstellen im Kristall bestimmt wird) berücksichtigt werden. Abhängig von der Tropfengröße, welche beispielsweise über eine stroboskopische Tropfenprojektion vermessen werden kann, ergibt sich für die Durchführung eines erfindungsgemäßen Verfahrens die benötigte Tropfenanzahl. Zur Ermittlung der Konzentration der Molekülspezies in der Lösung wird, da diese von der Anzahl der Proteinbindungsstellen im Kristall abhängig ist, die Annahme getroffen, dass die Proteinkristalle einen Wassergehalt von typischerweise ca. 50 % im Proteinkristall aufweisen. Unter Verwendung des Molekulargewichts des Proteins und unter Berücksichtigung der Bindungsstellen im Einzelprotein kann die Konzentration der Bindungsstellen im Kristall errechnet werden.

In der aufzubringenden Lösung kann nur eine Molekülspezies, d.h. eine Art eines Moleküls oder eines Molekülfragments enthalten sein, ebenfalls können aber auch verschiedene Moleküle oder Molekülfragmente und damit mehr als eine Molekülspezies in einer solchen Lösung enthalten sein. Es handelt sich dann um einen sog. "Cocktail"-Ansatz in der aufzubringenden Lösung. Hierbei sollten die miteinander kombinierten verschiedene Molekülspezies, vorzugsweise mindestens zwei, stärker bevorzugt mindestens drei, noch stärker bevorzugt mindestens zehn, noch stärker bevorzugt mindestens zwanzig und am stärksten bevorzugt mindestens fünfzig verschiedene Molekülspezies, eine vergleichbare Löslichkeit in dem gewählten Lösungsmittel(gemisch) aufweisen. Weiter ist es bevorzugt, dass in einem solchen Cocktail-Ansatz die einzelnen Molekülspezies nicht miteinander interagieren oder reagieren, sondern auch als solche monodispers jeweils weiter in der Lösung enthalten sind.

Vorteilhaft für die Zusammensetzung eines Fragmentcocktails, der im Rahmen der vorliegenden Erfindung eingesetzt wird, ist daher eine ähnliche Lösbarkeit der im Cocktail enthaltenen Fragmente. Insbesondere sollten die im Cocktail eingesetzten Fragmente nicht weniger als 1/5 der mittleren Löslichkeit bzw. nicht mehr als die 5-fache mittlere Löslichkeit der anderen im Cocktail enthaltenen Fragmente aufweisen. Vorteilhaft ist es weiterhin, wenn die Fragmente im Cocktail ihrer Struktur nach Unterschiede aufweisen bzw. sich bezüglich ihres Streuverhaltens unterscheiden, so dass röntgenkristallographisch die einzelnen, ggf. im Kristall gebundenen Fragmente eindeutig identifiziert werden können. Weiterhin bevorzugt ist es, wenn die im Cocktail enthaltenen Fragmente unterschiedliche physikochemische Eigenschaften aufweisen, um ausschließen zu können, dass die Fragmente um dieselbe Bindungsstelle im Zielprotein konkurrieren. Insoweit ist es vorteilhaft, wenn die Fragmente - stereochemisch voneinander abweichend - ein jeweils typisches Muster an funktionellen Merkmalen aufweisen, bspw. eine für jede Fragmentspezies charakteristische strukturelle Anordnung von ggf. verschiedenen funktionellen Gruppen oder Interaktionsparametern (bspw. eine aromatische Gruppe für Stapeleffekte ("stacking") oder Wasserstoffbrückenbindungsgruppen). Auf diese Weise kann mit Hilfe eines Fragmentcocktails im Rahmen eines erfindungsgemäßen Verfahrens eine möglichst große Anzahl an strukturell unterschiedlichen Ligandenfragmenten, die an verschiedene Bereiche innerhalb der Bindungstasche des Zielproteins binden können, getestet werden, wobei diese Fragmente Bestandteil eines auf deren Basis entwickelten, die Bindungstasche besetzenden Liganden sein können.

Darüber hinaus können die Fragmente, bspw. auch die Fragmente in einem Cocktailansatz, chemisch so modifiziert sein bzw. so gewählt werden, dass alle (oder zumindest ein Teil der Fragmente) die Eigenschaft besitzen, anomal Röntgenstrahlen zu streuen bzw. elektronenreiche Zentren aufzuweisen. Derartige elektronenreiche Zentren können bspw. Schwer(metall)atome (bspw. Kupfer-, Selen-, Quecksilber-, Goldatome) sein, die mit den Fragmenten derivatisiert werden bzw. an diese konjugiert sind. Auf diese Weise kann ein ohne derartige elektronenreiche Zentren nur schwach streuendes Fragment im Zielprotein röntgenkristallographisch ohne weiteres erkannt und topographisch zugeordnet werden. Diese Derivatisierung mit bspw. Schwermetallatomen wird insbesondere bei kleinen Fragmenten mit einem Molekulargewicht von weniger als 200 Da, insbesondere weniger als 100 Da bevorzugt sein, um die Fragmente röntgenkristallographisch erfassen zu können.

Schließlich kann das erfindungsgemäße Verfahren in einer weiteren bevorzugten Ausführungsform auch dazu eingesetzt werden, die Phasenbestimmung von solchen kristallisierten Zielproteinen zu ermöglichen, die strukturell noch nicht aufgeklärt wurden. Hierzu bedient man sich bspw. der Bestimmung der Phasen über anomale Streumethoden bzw. mit Hilfe von Schwermetallatomderivaten des Zielprotein. Derartige Schwermetallatome können als solche im Protein gebunden sein oder als Komplex im Protein vorliegen. Das erfindungsgemäße Verfahren erlaubt es, die Schwermetallatome oder deren Komplexe als Fragmente im Sinne der vorliegenden Erfindung einzusetzen und deren Bindung bzw. deren Bindungsort im Protein zu bestimmen und hierdurch die für die röntgenkristallographische Strukturaufklärung erforderliche Phaseninformation zu gewinnen. Derartige Schwermetallatome oder deren Komplexe können erfindungsgemäß auch im Cocktailansatz auf den Proteinkristall aufgebracht werden. Ein erfindungsgemäßes Verfahren eignet sich insbesondere deshalb zur systematischen Schwer(metall)atomderivatisierung, weil die Schwer(metall)atome oder deren Verbindungen oder Komplexe nur schwach (mit geringer Affinität) an die Zielproteine binden und/oder häufig nur schwerlöslich in wässrigen Lösungen sind. Gerade diese Nachteile werden aber durch das erfindungsgemäße Verfahren überwunden.

Ein besonderer Vorteil eines erfindungsgemäßen Verfahrens besteht weiterhin darin, dass mit Hilfe der Zugabe eines Cocktails verschiedener (Fragment)molekülspezies synergistische Effekte einzelner Spezies auf die Zielstruktur aufgedeckt und für die Identifizierung eines Liganden oder Inhibitors, der die einzelnen Fragmente, ggf. über Linker miteinander verbunden, enthält, entsprechend berücksichtigt werden können. Vielfach unterliegen die Zielproteine, für die Fragmente von Liganden oder Inhibitoren durch das erfindungsgemäße Verfahren identifiziert werden sollen, einer strukturellen Veränderung nach Bindung eines Liganden ("induced fit"). In solchen Fällen ist mit einer signifikanten Abhängigkeit der Bindungsmodi bei der gleichzeitigen Verwendung verschiedener aktiver Fragmente zu rechnen. Derartige strukturelle Abhängigkeiten können durch die Verwendung von Fragmentcocktails im Rahmen erfindungsgemäßer Verfahren systematisch untersucht werden. Regelmäßig ist als Folge des "induced fit" nach Bindung eines Fragments zu erwarten, dass eine Strukturveränderung im Protein eintritt und erst dann ein oder mehr weitere/s Fragment/e an das Protein binden können. Während die Zugabe größerer Liganden (enthaltend mehrere Fragment) auf Grund bspw. seiner strukturellen Starrheit keine Bindungsaffinität an die Proteine im Kristall erkennbar erscheinen läßt, wird die Bindung einzelner isolierter Fragmente des größeren Liganden möglich (auf Grund ihrer vergleichsweise geringen Größe können diese an die Zielstruktur binden, obwohl diese eine Strukturänderung durchmacht). Es lassen sich somit die durch den "induced fit" vorgegebenen Strukturveränderungen der Zielstruktur (und die damit gegenüber dem größeren Liganden zu modifizierenden Linker zwischen den Fragmenten) erkennen und für das Inhibitordesign berücksichtigen.

Ein erfindungsgemäßes Verfahren der vorstehenden Art kann Bestandteil des Verfahrens zur Ermittlung einer kristallographischen Struktur eines Komplexes von einer Target-Struktur, beispielsweise eines Proteins, und von mindestens einer Molekülspezies sein. In einem solchen Verfahren wird zunächst das vorbeschriebene erfindungsgemäße Verfahren mit seinen Verfahrensschritten durchgeführt und danach oder zugleich wird auf dem Fachmann bekannte Weise der verfahrens- und erfindungsgemäß behandelte Kristall mit Röntgenstrahlung oder Synchrotronstrahlung bestrahlt, so dass schließlich das Beugungsbild des Kristalls aufgenommen werden kann, d.h. die Datensammlung der im Beugungsbild auftretenden Reflexe erfolgen kann.

Aus der Datensammlung, d.h. den Intensitäten der beobachteten Reflexe, kann dann in einem weiteren Verfahrensschritt eine Elektronendichtekarte errechnet werden, wobei hierfür Phaseninformation benötigt wird. Diese Phaseninformation kann durch andere Techniken bereitgestellt werden, bspw. durch Schwermetallatomderivate, die die Phaseninformation verschaffen ("isomorphous replacement") oder durch Methoden des "multiple anomolous scattering (MAD)" (Stout und Jensen, 1989, John Wiley, New York). Besonders bevorzugt sind allerdings die Verfahren des "molecular repiacement", da ein erfindungsgemäßes Verfahren regelmäßig mit an sich bekannten dreidimensionalen Strukturen von Targets durchgeführt wird und daher die Phaseninformation der Targetstruktur (ohne die verfahrensgemäß aufgetropften Liganden) bereits verfügbar ist. Besonders bevorzugt kann die Positionierung der gebundenen Moleküle oder Molekülfragmente, also der Testsubstanzen mit Liganden-Eigenschaft, in der Targetstruktur dann ermittelt werden, wenn eine Differenzelektronendichtekarte errechnet wird. Hierzu wird die Differenz in der Elektronendichte zwischen komplexierter und nicht-komplexierter Targetstruktur ermittelt, wobei die verbliebene Elektronendichte gerade der Elektronendichte des oder der in einer Targetstruktur gebundenen Moleküls/e entspricht.

Der Beschuß des Kristalls mit Röntgenstrahlung kann bereits während des Auftropfens erfolgen oder nach Abschluß des Auftropfens. Bevorzugt ist insbesondere die Verwendung von "weißer Röntgenstrahlung", also bspw. von Synchrotronstrahlung während des Auftropfens der Molekülspezies. Auf diese Weise kann die sukzessive Besetzung der Bindungsplätze für den oder die Liganden in der Targetstruktur im Kristall verfolgt werden. Besonders vorteilhaft ist es daher, wenn ein Verfahren zur Identifikation von ein kristallisiertes Protein bindenden Molekülen eingesetzt wird, bei dem (a) eine Molekülspezies nach einem Verfahren nach einem der Ansprüche 1 bis 24 auf den Kristall aufgebracht wird, (b) in einem zeitlichen Abstand von variabler Länge Beugungsintensitäten gemessen werden und (c) diese im zeitlichen Abstand gemessenen Beugungsintensitäten entsprechend ihrer zeitlichen Abfolge miteinander verglichen werden. Hierbei ist es besonders bevorzugt, wenn der Kristall in einer Orientierung während aller Beugungsaufnahmen verbleibt. Auf diesem Weg wird es erfindungsgemäß möglich mit nur einem Kristall und einzelnen Röntgenaufnahmen (ohne einen vollständigen Datensatz aufnehmen zu müssen), die Komplexbildung nachzuweisen und damit die Testsubstanz als Liganden oder als nicht-bindend zu identifizieren. Mit zunehmender Komplexbildung nimmt nämlich die Korrelation zum völlig unbesetzten Ausgangszustand des Kristalls ab, weswegen (im zeitlichen Abstand wachsende) Intensitätsunterschiede des Reflexe die Komplexbildung indizieren. Ein derartiges Verfahren kann als Hochdurchsatzverfahren durchgeführt werden, da eine nichtbindende Substanz verworfen werden kann und mit einer anderen Substanz das Verfahren gemäß Schritten (a) bis (c) wiederholt werden kann. Innerhalb von wenigen Minuten kann erfindungsgemäß eine Testsubstanz als Ligand identifiziert oder als nichtbindend verworfen werden.

Ein erfindungsgemäßes Verfahren kann auch mit weiteren vor- oder nachgeschalteten Verfahrensschritten kombiniert werden. Insbesondere können nach Lokalisierung von mindestens einem Fragment als Ligand einer Targetstruktur, vorzugsweise von mindestens zwei an benachbarten Positionen in der Targetstruktur bindenden Fragmenten oder kleinen Molekülen, die Fragmente durch Konstruktion eines chemischen Linkers, der den Vorgaben der Targetstruktur entspricht, miteinander verbunden werden und ein Molekül chemisch synthetisiert werden, das die mindestens zwei durch das erfindungsgemäße Verfahren identifizierten Fragmente miteinander kovalent verknüpft. Ein derartiger aus Fragmenten kombinierter Ligand sollte zur Targetstruktur eine entsprechend erhöhte Bindungsaffinität aufweisen. Die Ausgestaltung eines Linkers kann bspw. über etablierte computerexperimentells Methoden erfolgen, bspw. über LigBuilder (Journal of Molecular Modeling 2000, 6, 498) und spezielle Funktionen der vorgenannten Programme LUDI, SPROUT, GROW, PROLIGAND oder LEAPFROG. Die Bindung des mit Hilfe eines oder mehrerer Linker verknüpften Liganden kann dann wiederum röntgenkristallographisch untersucht und die Eignung des jeweiligen Linkers, der bspw. auch flexibel mit zahlreichen rotierbaren chemischen Bindungen versehen sein kann, zur Kombination der Fragmente überprüft werden. Sollte in einer bevorzugten Ausführungsform ein sehr flexibler Linker in einem ersten Ansatz eingesetzt worden sein, kann die im Kristall auftretende Struktur des Linkers ermittelt und in einem weiteren Ansatz die Flexibilität des Linkers nach Maßgabe der Positionierung des flexiblen Linkers in der Targetstruktur eingeschränkt werden, um derart die intrinsische Paßgenauigkeit des Ligangen zu erhöhen.

Bevorzugt kann vor der Durchführung des erfindungsgemäßen Verfahrens eine Vorauswahl der in der auf den Kristall aufzubringenden Lösung enthaltenen Fragmente erfolgen. Auf diese Weise kann die Zahl der potentiell an die Zielstruktur bindenden Fragmente in der aufzubringenden Lösung erhöht werden. Eine Möglichkeit eine Vorauswahl zu treffen, besteht in der Vorschaltung eines computerexperimentellen Verfahrensschritts. Derart wird durch entsprechende Progamme die erwünschte Bindungsregion auf der Zielstruktur analysiert und gemäß den sterischen oder funktionellen Randbedingungen der die Bindungstasche bildenden Aminosäuren eine Vorhersage für einen geeigneten Liganden bzw. dessen Teilstruktur erhalten. Derartige Programme (bspw. LigBuilder, LEAPFROG, GROW, LUDI, SPROUT, PROLIGAND, s.o.) können eingesetzt werden, um zielgerichtet Fragmente für die nachfolgende Durchführung des erfindungsgemäßen Verfahrens auszuwählen ("in silico Docking" Verfahren). Eine weitere Möglichkeit, vor der Durchführung des erfindungsgemäßen Verfahrens vermeintlich bindende Fragmente zu identifizieren, stellen in vitro Testsysteme dar. Hierbei kommen geeignete Testsysteme in Betracht, die auch bei nur schwach bindenden Fragmenten eine Unterscheidung zwischen nicht-bindenden und schwach bindenden Fragmenten erlauben. Bspw. kann ein derartiger in vitro Test mit Hilfe von Adsorptionssäulen durchgeführt werden. Die Zielstruktur wird auf der Adsorptionssäule gekoppelt und daran gebundene Fragmente isoliert und identifiziert. Ganz besonders bevorzugt können für in vitro Testversuche Bibliotheken von Fragmenten eingesetzt werden, bspw. auf der Basis von Naturstoffen, bspw. Peptidbibliotheken von bspw. Dipeptiden, ggf. als Peptidomimetika, bspw. als Gerüstpeptidomimetika. Schließlich können als in vitro "Screening"-Methoden zur Ermittlung einer Vorauswahl von Fragmenten auch biophysikalische Methoden zum Einsatz kommen, wie z.B. NMR-Spektroskopie oder Oberflächenplasmonresonanzspektroskopie (z.B. nach dem Verfahren von Biacore). Beide spektroskopischen Methoden können schwach bindende Fragmente identifizieren und eignen sich somit als Verfahrensschritt vor dem erfindungsgemäßen Verfahren.

Ein weiterer Gegenstand der vorliegenden Erfindung ist ein Verfahren zur Identifikation von einem eine Zielstruktur bindenden Liganden, wobei (a) ein Verfahren nach einem der Ansprüche 1 bis 24 durchgeführt wird, (b) nach einem Verfahren nach einem der Ansprüche 25 bis 29 die Struktur von mindestens einem Komplexes mit mindestens zwei Fragmenten ermittelt wird, (c) Linker zwischen den mindestens zwei Fragmenten zu einem Liganden ermittelt wird/werden und (d) ein Ligand, enthaltend die mindestens zwei Fragmente und den mindestens einen Linker, synthetisiert wird. Hierzu werden die vorgenannten verfahren eingesetzt, d.h. die Fragmente werden durch Linker, die bspw. auf Grund der Proteinstruktur ermittelt werden (bspw. durch das Programm LigBuilder), miteinander verbunden und entsprechende Verbindungen synthetisiert. Mit diesen aus den Fragmenten baukastenartig aufgebauten Verbindungen (Liganden), die eine sehr viel höhere Affinität zur Zielstruktur aufweisen als die einzelnen Fragmente, können dann wiederum Strukturuntersuchungen durchgeführt werden bzw. diese können in entsprechenden Assays auf ihre biologische Wirksamkeit hin untersucht werden.

Das erfindungsgemäße Verfahren wird durch eine Vorrichtung zum Behandeln eines Kristalls mit einer Flüssigkeit (Lösung) mit einer Halterung zur Befestigung des Kristalls und einem Mikrodosiersystem möglich, das im Verhältnis zur Halterung so angeordnet ist, daß damit Mikro-Tropfen einer Flüssigkeit, die bspw. Lösungsmittel und mindestens einen Ligandentyp aufweist, auf den in der Halterung befestigten Kristall aufgebracht werden können.

Die zur Durchführung des erfindungsgemäßen Verfahrens eingesetzte Vorrichtung kann in verschiedenen vorteilhaften Ausführungsformen vorliegen.

In einer vorteilhaften Ausführungsform der zur Durchführung des erfindungsgemäßen Verfahrens geeigneten Vorrichtung weist diese darüber hinaus ein Mittel auf, mit dem während des Auftropfens der Flüssigkeit um den Kristall herum eine definierte Umgebung erzeugt werden kann. In einer weiteren vorteilhaften Ausführungsform der zur Durchführung des erfindungsgemäßen Verfahrens geeigneten Vorrichtung besteht das Erzeugen der definierten Umgebung darin, einen Gasstrom definierter Zusammensetzung um den Kristall herum zu erzeugen. In noch einer weiteren vorteilhaften Ausführungsform der zur Durchführung des erfindungsgemäßen Verfahrens geeigneten Vorrichtung ist die Halterung darüber hinaus so ausgebildet ist, daß durch die Halterung der Gasstrom so geführt werden kann, daß er auf den in der Halterung befestigten Kristall gerichtet ist. Dadurch kann der Kristall während der Behandlung durch die Mikro-Tropfen in einer definierten Umgebung gehalten werden.

Bei einer Vorrichtung zur Durchführung eines erfindungsgemäßen Verfahrens kann vorteilhafterweise die Halterung aus einem Trägerblock für eine Haltekapillare bestehen, die ein freies Auflageende für den Kristall besitzt. Schließlich kann daran anknüpfend die Haltekapillare aus einer Mikropipette bestehen, in der sich ein Unterdruck erzeugen läßt, um den Kristall zu halten. Ferner kann bei einer vorteilhaften Ausrührungsform der Trägerblock der Halterung einen integralen Gaskanal mit einem Mündungsende enthalten, das auf das Auflageende der Haltekapillare gerichtet ist.

In einer weiteren vorteilhaften Ausführungsform der zur Durchführung des erfindungsgemäßen Verfahrens geeigneten Vorrichtung kann diese darüber hinaus ein Gasmischmittel aufweisen, mit dem die Zusammensetzung des Gasstroms variabel eingestellt werden kann. Bei einer solchen vorteilhaften Ausführungsform kann das Gas aus Luft mit einem bestimmten Feuchtigkeitsgehalt bestehen und das Gasmischmittel so ausgebildet sein, daß damit die Luftfeuchtigkeit eingestellt werden kann. Auch kann eine solche Vorrichtung darüber hinaus ein Lösungsvermittler-beigabemittel umfassen, mit dem dem Gasstrom ein Lösungsvermittler für eine in die Kristallstruktur des Kristalls einzubringende Substanz beigemischt werden kann. Weitergebildet werden kann eine solche Vorrichtung dadurch, dass sie bevorzugt darüber hinaus ein Konzentrationseinstellmittel zur Einstellung der Konzentration des Lösungsvermittlers umfaßt.

Eine Vorrichtung zur Durchführung eines erfindungsgemäßen Verfahrens kann darüber hinaus ein Temperatureinstellmittel umfassen, mit dem die Temperatur des Gasstroms variabel eingestellt werden kann.

In einer weiteren vorteilhaften Ausführungsform der zur Durchführung des erfindungsgemäßen Verfahrens geeigneten Vorrichtung ist deren Mikrodosiersystem so ausgebildet, daß es Mikro-Tropfen der auf den Kristall aufzubringenden Flüssigkeit erzeugen kann, die ein Volumen aufweisen, das kleiner als das Volumen des Kristalls ist. Bevorzugt ist hierbei, dass bei der Vorrichtung das Mikrodosiersystem so ausgebildet ist, daß es Mikro-Tropfen erzeugen kann, deren Volumen zwischen 10 und 20 Prozent des Kristallvolumens und vorzugsweise zwischen 5 und 10 Prozent des Kristallvolumens beträgt. Vorteilhafterweise ist bei einer solchen Vorrichtung das Mikrodosiersystem so ausgebildet, daß es Mikro-Tropfen erzeugen kann, deren Volumen zwischen 1 nl und 100 pl, vorzugsweise zwischen 100 pl und 20 pl und noch bevorzugt zwischen 20 pl und 4 pl liegt.

In einer weiteren vorteilhaften Ausführungsform der zur Durchführung des erfindungsgemäßen Verfahrens geeigneten Vorrichtung weist das Mikrodosiersystem darüber hinaus ein Flüssigkeitszufuhrsystem auf, mit dem verschiedene Flüssigkeiten, die auf den Kristall aufgetropft werden sollen, in zeitlich gesteuerter Weise einem Tropfenerzeugungsteil des Mikrodosiersystems zugeführt werden können. Bei einer derartigen Vorrichtung umfaßt das Flüssigkeitszufuhrsystem des Mikrodosiersystems eine elektrisch ansteuerbare Präzisionsspritze und ein Leitungssystem, mit dem die Präzisionsspritze über elektrisch steuerbare Ventile mit verschiedenen Flüssigkeitsvorratsbehältern und mit dem Tropfenerzeugungsteil des Mikrodosiersystems verbunden werden kann, um diesem Flüssigkeit für die Tropfenerzeugung zuzuführen.

In einer weiteren vorteilhaften Ausführungsform der zur Durchführung des erfindungsgemäßen Verfahrens geeigneten Vorrichtung ist das Mikrodosiersystem so ausgebildet, daß es eine Piezopipette umfaßt, die das Tropfenerzeugungsteil bildet. Vorteilhaft ist es hierbei, wenn bei einer solchen Vorrichtung die Piezopipette aus einer Kapillare besteht, die von einem piezoelektrischen Element umschlossen ist. Schließlich kann bei einer solchen Vorrichtung das Mikrodosiersystem darüber hinaus ein mit der Piezopipette elektrisch verbundenes Steuergerät umfassen, das so ausgebildet ist, daß damit unterschiedlich geformte Spannungspulse an die Piezopipette angelegt werden können, deren Formen die Form und Größe der Mikro-Tropfen und deren Frequenz die Frequenz der Mikro-Tropfen steuern.

Vorteilhaft ist eine Vorrichtung zur Durchführung eines erfindungsgemäßen Verfahrens auch dann, wenn bei dieser das Mikrodosiersystem eine Kapillare und ein in der Kapillare angeordnetes Mikroventil umfaßt. Schließlich kann das Mikrodosiersystem einer solchen Vorrichtung darüber hinaus ein Steuergerät zum Ein- und Ausschalten des Mikroventils umfassen, um die Mikro-Tropfen zu erzeugen.

Verwendet wird eine Vorrichtung zur Durchführung eines erfindungsgemäßen Verfahrens besonders bevorzugt dann, wenn sie mehrere Mikrodosiersysteme umfaßt, die im Verhältnis zur Halterung so angeordnet sind, daß damit Mikro-Tropfen verschiedener Flüssigkeiten auf den in der Halterung befestigten Kristall aufgebracht werden können. Schließlich sollte bei einer solchen Vorrichtung die Halterung darüber hinaus so ausgebildet sein, daß sie zur Befestigung eines Proteinkristalls geeignet ist.

Bevorzugt besteht bei einer Vorrichtung die verwendete Flüssigkeit aus einer Lösung, wie vorstehend bei den bevorzugten Ausführungsformen eines erfindungsgemäßen Verfahrens offenbart.

Bevorzugt sind weiterhin Vorrichtungen, bei denen in der Lösung eine Substanz oder mehrere Substanzen gelöst ist bzw. sind, die in die Struktur des Kristalls eingebracht werden soll bzw. sollen oder mit dieser reagieren soll bzw. sollen. Bei derartigen Vorrichtungen kann/können die Substanz bzw. die Substanzen aus einem oder mehreren Liganden oder Inhibitoren bestehen.

Es ist ferner besonders vorteilhaft, daß die erfindungsgemäße Vorrichtung auch auf einem Goniometerkopf im Röntgenstrahl oder in einem Synchrotron befestigt werden kann, so daß der zeitliche Ablauf der Veränderung der kristallisierten Proteinstruktur, z.B. infolge der Ligandenbindung während des Auftropfens der Mikro-Tropfen, im Meßgerät beobachtet werden kann. Damit kann ein Goniometerkopf eine Vorrichtung, wie vorstehen offenbart, zur Durchführung eines erfindungsgemäßen Verfahrens aufweisen. Ein solcher Goniometerkopf mit einer derartigen Vorrichtung kann wiederum Bestandteil einer Röntgenbestrahlungsanlage oder einer Synchrotronbestrahlungsanlage sein.

Damit werden vorliegendenfalls die vorbeschriebenen Ausführungsformen einer Vorrichtung zur Durchführung eines erfindungsgemäßen Verfahrens, wie es in dieser Anmeldung sich mit seinen bevorzugten Ausgestaltungsformen offenbart findet, ebenfalls offenbart.

Besonders bevorzugte Ausführungsformen der zur Durchführung des erfindungsgemäßen Verfahrens geeigneten Vorrichtung werden nachfolgend anhand der Figuren näher erläutert.

Es zeigen
Fig. 1 eine teilweise im Schnitt dargestellte Ansicht einer Ausführungsform einer erfindungsgemäßen Vorrichtung zum Behandeln eines Kristalls mit einer Lösung,
Fig. 2 ein Gehäuse eines Steuergeräts zur Steuerung eines bei einer Ausführungsform der erfindungsgemäßen Vorrichtung verwendeten Mikrodosiersystems,
Fig. 3 zeigt ein Flüssigkeitszufuhrsystem für ein Mikrodosiersystem, das bei einer Ausführungsform der erfindungsgemäßen Vorrichtung verwendet werden kann.

Die Erfindung wird im folgenden am Beispiel der Behandlung von Proteinkristallen beschrieben, sie kann aber auch in analoger Weise bei der Behandlung von anderen Kristallen eingesetzt werden.

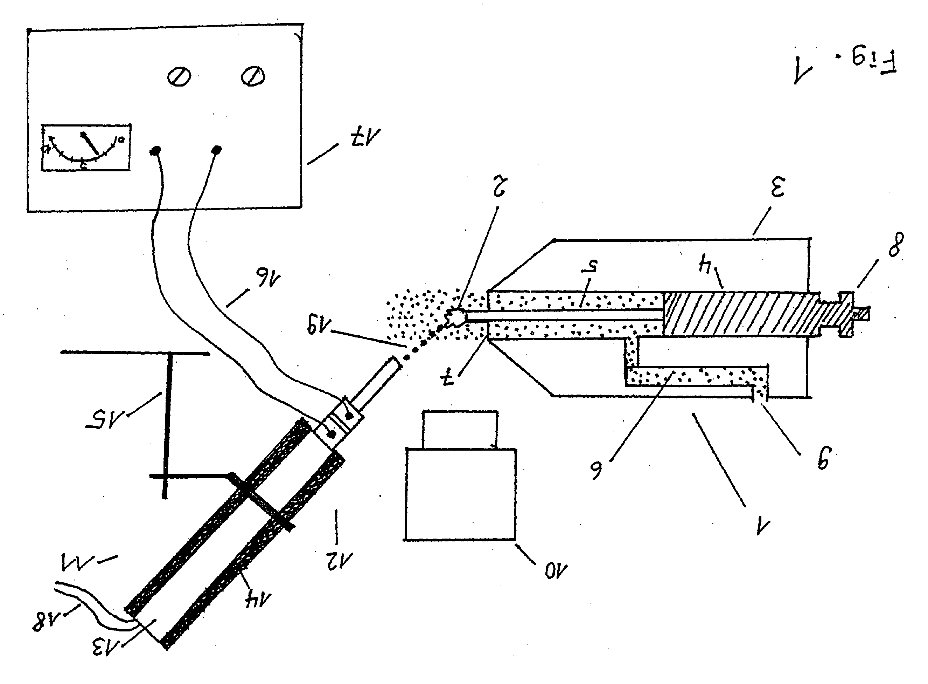

Fig. 1 zeigt eine erste Ausführungsform einer erfindungsgemäßen Vorrichtung zum Behandeln eines Kristalls. Dabei ist links in der Fig. 1 eine Halterung 1 dargestellt, die dazu dient, einen Proteinkristall 2 zu befestigen. Die in der Fig. 1 dargestellte Halterung, die in ihrer generischen Art auch als "free mounting system" bezeichnet wird, ist bereits aus dem Stand der Technik bekannt und z.B. in der Deutschen Patentschrift DE 198 42 797 C1 beschrieben worden. Diese Druckschrift wird insoweit vollumfänglich in die Offenbarung der vorliegenden Anmeldung einbezogen.

Die Halterung 1, die in der Fig. 1 in einer Schnittansicht von der Seite dargestellt ist, besteht im wesentlichen aus einem Trägerblock 3, der ein einschiebbares Einsatzteil 4 aufweist, das in eine Öffnung des Trägerblocks 3 eingeschoben werden kann. Am Einsatzteil ist eine Haltekapillare 5 angebracht, an deren freiem Auflageende der Proteinkristall 2 gehalten wird. Die Haltekapillare besteht vorzugsweise aus einer Mikropipette, in der über eine in der Fig. 1 nicht dargestellte und mit dem anderen Ende der Mikropipette verbundene Pumpvorrichtung ein Unterdruck erzeugt wird, der dazu dient, den Proteinkristall 2 an dem freien Auflageende zu halten. Das linke Ende 8 des Einsatzteils ist so ausgebildet, daß daran die Halterung 1 an einem Goniometerkopf einer Röntgen- oder Synchrotronbestrahlungsanlage befestigt werden kann.

In einer Röntgen- oder Synchrotronbestrahlungsanlage kann die Beugung von Röntgenstrahlen beim Durchgang durch das Kristallgitter des Proteinkristalls ausgenutzt werden, um aus dem Beugungsbild auf die räumliche Anordnung der Atome und Moleküle in dem kristallisierten Protein zu schließen bzw. die Struktur durch mathematische Operationen zu errechnen. Die erforderlichen Röntgenstrahlen können z.B. durch Beschuß von Kupfer oder anderen Materialien mit Elektronen erzeugt werden (bspw. CuKα-Strahlung). Alternativ kann die Röntgenstrahlung auch in einem Synchrotron, d.h. einem Teilchenbeschleuniger, erzeugt werden, bei dem die Röntgenstrahlung von auf Kreisbahnen beschleunigten Elektronen emittiert wird. Das Synchrotron besitzt trotz des größeren apparativen Aufwands eine Reihe von Vorteilen gegenüber der herkömmlichen Erzeugung von Röntgenstrahlung durch Elektronenbeschuß von Metallen. So besitzen die durch Synchrotrone erzeugten Röntgenstrahlen eine höhere Intensität und können in verschiedenen Wellenlängen gewählt werden. Auch besteht auf diese Weise die Möglichkeit, "weißes" Röntgenlicht einzusetzen und damit den Kristall mit Röntgenblitzen, die Röntgenstrahlen aller Wellenlängen aufweisen, zu beschießen. Darüber hinaus lassen sich die Messungen mit dem Synchrotron wesentlich schneller als mit herkömmlichen Röntgenbestrahlungsanlagen durchführen.

In die Halterung 1 ist ferner ein Gaskanal 6 integriert, dessen Mündungsende 7 auf das freie Auflageende der Haltekapillare 5 gerichtet ist, an dem der Proteinkristall 2 befestigt ist. Dabei wird der am Auflageende angebrachte Proteinkristall 2 vollständig vom Gasstrom aus dem Gaskanal 6 umschlossen, so daß eine definierte Gasatmosphäre um den Proteinkristall herum erzeugt werden kann. Der Gaskanal 6 ist an seinem in der Fig. 1 als offen dargestellten Ende mit einem Gaserzeugungsmittel und einem Gasmischmittel verbunden, mit dem die Zusammensetzung des Gasstroms variabel eingestellt werden kann. Falls das um den Proteinkristall herum befindliche Gas Luft ist, kann das Gasmischmittel z.B. dazu dienen, die Luftfeuchtigkeit auf einen vorherbestimmten optimalen Wert einzuregeln. Es kann darüber hinaus auch ein Temperatureinstellmittel vorgesehen sein, mit dem die Temperatur des Gasstroms gemessen und auf einen bestimmten vorgebbaren Wert eingeregelt werden kann. Auch können andere gasförmige Substanzen dem Gasstrom beigemischt werden, so dass bspw. der Stickstoff- oder Sauerstoffgehalt der Luft modifiziert, bspw. erhöht, werden kann.

In der Deutschen Patentanmeldung Nr. 10232172.8-52 mit dem Titel "Vorrichtung und Verfahren zur Erzeugung einer definierten Umgebung für partikelförmige Proben" ist bereits eine Vorrichtung und ein Verfahren beschrieben, mit dem sich eine hochgenaue und langzeitstabile Feuchteeinstellung eines durch die oben beschriebene Halterung geführten feuchten Gasstroms am Ort des partikelförmigen Kristalls erreichen läßt. Diese Druckschrift wird daher insoweit ebenfalls vollumfänglich in die Offenbarung der vorliegenden Anmeldung einbezogen.

Über dem Kristall ist ein Mikroskop mit Videosystem 10 angebracht, mit dem der Proteinkristall während der Behandlung mit der Substanz beobachtet werden kann. Ggf. kann infolge der Beobachtung über das Videosystem der Behandlungsmodus modifiziert oder auch die Behandlung eingestellt werden.

Die in der Fig. 1 dargestellte erfindungsgemäße Vorrichtung zum Behandeln eines Kristalls mit einer Substanz umfaßt darüber hinaus ein Mikrodosiersystem 11, das rechts in der Fig. 1 in einer Seitenansicht im Schnitt dargestellt ist.

Das Mikrodosiersystem 11 umfaßt eine sogenannte Piezopipette 12, die in einem Stativ 15 gehalten wird und so auf den Proteinkristall 2 ausgerichtet ist, daß dieser mittels der Piezopipette mit Tropfen beschossen werden kann. Die Piezopipette ist in der Fig. 1 aus Gründen der Anschaulichkeit in einem vergrößerten Maßstab im Verhältnis zur Halterung 1 dargestellt. Die Piezopipette ist so angeordnet, daß die Spitze der Piezopipette einen Abstand von typischerweise 3 mm zu dem Proteinkristall aufweist. Vorzugsweise liegt dieser Abstand in einem Bereich von 1 - 5 mm, kann jedoch unter besonderen Umständen auch größer oder kleiner gewählt werden.

Die Piezopipette 12 besteht aus einer Glaskapillare 13, die z.B. aus Borosilicatglas bestehen kann. Die Durchmesser der Öffnung der Glaskapillare ist einer der Faktoren, die die Größe der von der Piezopipette abgegebenen Mikro-Tropfen beeinflussen und kann z.B. in einem Bereich zwischen 5 und 50 Mikrometer liegen. Die Glaskapillare 13 ist von einem piezoelektrischen Element 14 umschlossen, das aus einem Material besteht, das einen piezoelektrischen Effekt zeigt. Es kann sich bei diesem Material z.B. um einen Piezokristall handeln. Das piezoelektrische Element 14 ist darüber hinaus über zwei Kabel 16 mit einem Steuergerät 17 elektrisch verbunden, mit dem eine Spannung an das piezoelektrische Element 14 angelegt werden kann. Wird ein Spannungspuls durch das Steuergerät 17 an das piezoelektrische Element 14 angelegt, so wird das piezoelektrische Element 14 und mit diesem auch die Glaskapillare 13 kontrahiert und ein Tropfen aus der Öffnung der Piezopipette herausgeschossen. Über das Steuergerät 17 können unterschiedlich geformte Spannungspulse an die Piezopipette angelegt werden können, deren Formen die Form und Größe der Mikro-Tropfen und deren Frequenz die Frequenz der Mikro-Tropfen beeinflussen.

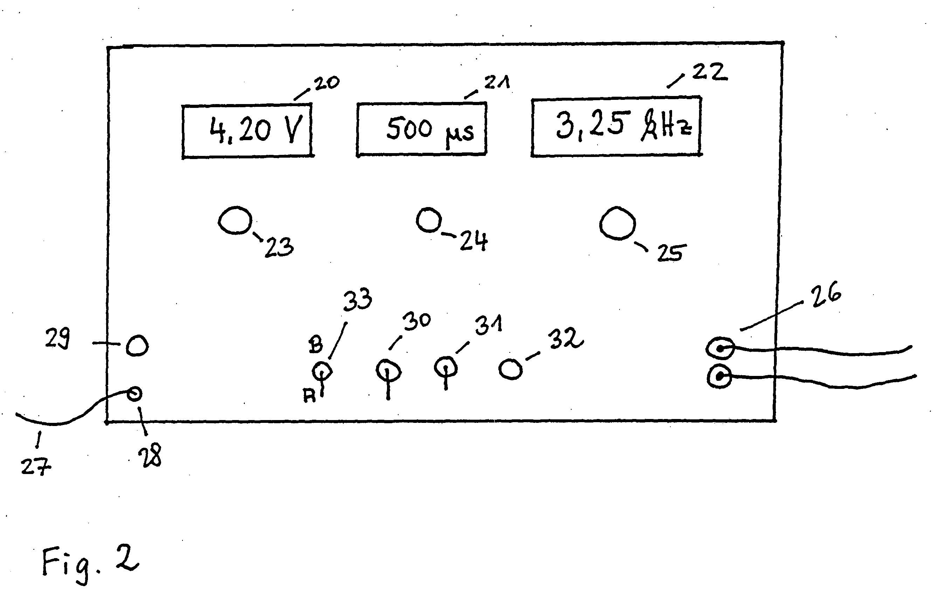

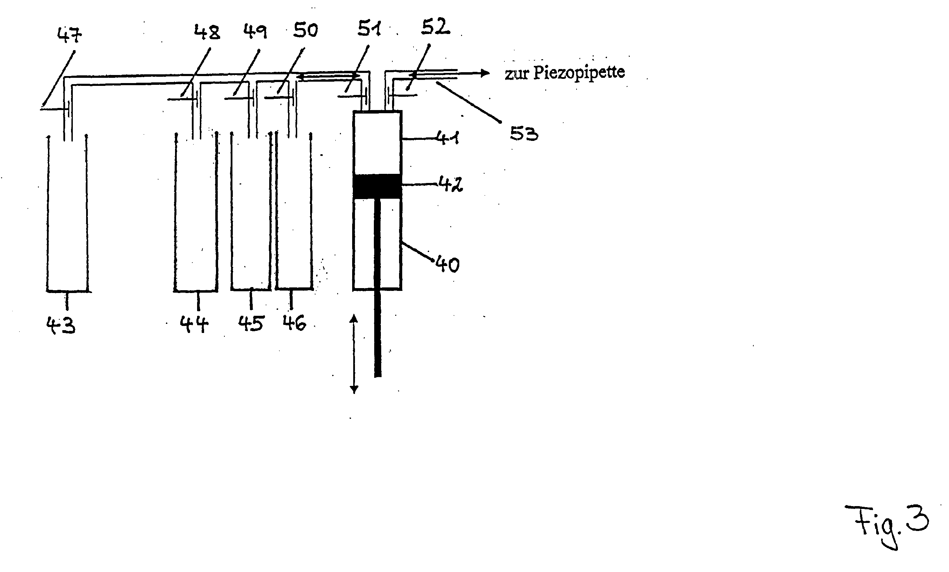

In der Fig. 2 ist ein Gehäuse eines möglichen Steuergeräts zur Steuerung der Piezopipette dargestellt, wobei die einzelnen Steuerungsmöglichkeiten anhand der in der Fig. 2 dargestellten Schalter und Steuerelemente des Steuergeräts erläutert werden sollen. Das Steuergerät weist zunächst drei verschiedene LCD-Anzeigen 20, 21 und 22 auf. Auf der ersten LCD-Anzeige 20 wird der aktuelle Wert für den Spannungspegel der Impulsausgangsspannung für das Piezopipettensteuersignal angezeigt. Dieser Wert läßt sich über einen Drehregler 23 variabel einstellen. Auch die Impulsweite des Pipettenansteuersignals, die auf der zweiten LCD-Anzeige 21 in Mikrosekunden angezeigt wird, läßt sich mittels eines zweiten Drehreglers 24 einstellen. Schließlich ist ein dritter Drehregler 25 vorgesehen, um die Frequenz der an die Piezopipette angelegten Spannungsimpulse einzustellen, die auf der dritten LCD-Anzeige 22 angezeigt wird. Diese Frequenz, die bis zu einige kHz betragen kann (z.B. 2 kHz) entspricht der Frequenz, mit der die Mikro-Tropfen aus der Piezopipette auf den Kristall geschleudert werden. Der Einstellbereich der Frequenz kann z.B. in einem Bereich zwischen 1 Hz und 6 kHz liegen. Die Höhe der Impulsausgangsspannung und die Weite der Spannungsimpulse müssen zunächst so eingestellt werden, daß es überhaupt zu einer Tropfenerzeugung mit der Piezopipette kommt. Darauf wird die Frequenz gewählt, die für den jeweiligen Kristallbehandlungsprozeß ideal ist. Die Frequenz kann natürlich auch während des Kristallbehandlungsprozesses laufend variiert werden.

Das Steuergerät weist ferner zwei Eingänge 26 auf, an denen die beiden Verbindungskabel der Piezopipette angeschlossen werden. Ferner sind ein Netzkabel 27 sowie ein Netzanschluß 28 zur Stromversorgung des Steuergeräts vorgesehen. Über den weiteren Signaleingang 29 können von anderen elektrischen Geräten vorgegebene Spannungsimpulsfolgen angelegt werden, um die Mikro-Tropfenerzeugung auszulösen und die Mikro-Tropfenfolge und -form von außen zu steuern. Das kann z.B. sinnvoll sein, wenn es ein zentrales Steuergerät gibt, das sowohl die Tropfenerzeugung als auch andere Parameter der Kristallbehandlung wie den über die Kristallhalterung zugeführten Gasstrom, die Zusammensetzung des Gasstroms (z.B. seinen Feuchtegehalt), die Temperatur des Gasstroms, eine angeschlossene Röntgenbestrahlungsanlage etc. steuert und die verschiedenen Steuerungsparameter in einer vorherbestimmten Weise zueinander synchronisiert.

Der Schalter 30 ist dazu vorgesehen, den Piezopipettenbetrieb ein- und auszuschalten. Über den weiteren Schalter 31 kann zwischen Einzelspannungsimpulsbetrieb und kontinuierlichem Spannungsimpulsbetrieb umgeschaltet werden, d.h. zwischen Einzeltropfenerzeugung und kontinuierlicher Tropfenerzeugung. Für die Einzeltropfenerzeugung kann ferner ein Taster 32 vorgesehen sein, über den einzelne Spannungsimpulse an die Piezopipette angelegt werden können, wenn es gewünscht ist, einzelne Tropfen per Handbetrieb auf den Kristall zu schießen.

Der Schalter 33 dient schließlich dazu zwischen verschiedenen Impulsformen der an die Piezopipette 12 angelegten Spannungsimpulse variieren zu können. In der Schalterstellung A kann z.B. ein vorgegebener Standard-Rechteckspannungsimpuls mit vorherbestimmter Dauer und Höhe erzeugt werden, während in der Schalterstellung B ein Rechteckspannungsimpuls erzeugt werden kann, dessen Dauer und Höhe variabel eingestellt werden kann. Es ist natürlich bei anderen Ausführungen auch denkbar, daß Spannungsimpulse angelegt werden, die von der Rechteckform abweichen. Die Impulsform der Spannungsimpulse wird nun so gewählt, daß eine optimale Tropfenerzeugung in Hinblick auf den zu behandelnden Kristall gewährleistet ist.

Verschiedene Größen der Mikro-Tropfen, die z.B. für verschiedene Kristallgrößen geeignet sein können, können über die Variation der Spannungspulsweiten und Spannungspulshöhen eingestellt werden, die die an die Piezopipette angelegten Spannungen aufweisen.

Die Glaskapillare 13 der Piezopipette 12 ist typischerweise über eine Zuleitung 18 mit einem in der Figur 1 nicht dargestellten Vorratsgefäß verbunden, das die Lösung enthält, die auf den Proteinkristall getropft werden soll. Diese Lösung enthält die Substanz oder die Substanzen, mit der bzw. denen der Proteinkristall behandelt werden soll. Die Oberkante des Flüssigkeitsspiegels der sich im Vorratsgefäß befindenden Flüssigkeit sollte dabei etwas höher als die Unterkante der Pipettendüse eingestellt werden. Alternativ dazu kann die Flüssigkeit bei einer Ausführungsform ohne Vorratsgefäß aber auch direkt über die Auslaßöffnung der Piezopipette in die Piezopipette gesaugt werden, um sie dann später wieder abgeben zu können. Es kann auch eine Temperiervorrichtung um das Vorratsgefäß herum angeordnet sein, um die in dem Vorratsgefäß sich befindende Flüssigkeit auf eine gewünschte Temperatur zu bringen. Gemäß einer Ausführungsform kann vor dem Aufbringen der Lösung auf den Kristall der pH-Wert und/oder die Ionenstärke (bzw. spezifische Salzkonzentrationen) der Lösung gemäß den im Stand der Technik bekannten Verfahren auf einen gewünschten Wert eingestellt werden.

Unter Mikro-Tropfen im Sinne der vorliegenden Erfindung sollen Tropfen zu verstehen sein, deren Volumen kleiner als 1 nl ist, wobei das Volumen der Mikro-Tropfen vorzugsweise zwischen 1 nl (nanoliter) und 1 pl (picoliter), noch weiter bevorzugt zwischen 100 pl und 20 pl und noch stärker bevorzugt zwischen 20 pl und 4 pl liegt. Aus diesen Größen lassen sich über die Volumensformel die entsprechenden geeigneten Durchmesser der Tropfen errechnen, wenn man näherungsweise davon ausgeht, daß die Tropfen kugelförmig sind. Die gewünschte Tropfengröße kann erfindungsgemäß eingestellt werden.

Die Mikro-Tropfen der auf den Kristall aufzubringenden Flüssigkeit sind dabei vorzugsweise kleiner als das Volumen des Kristalls ist. Ein typisches Kristallvolumen kann dabei z.B. in einer Großenördnung von 1 nl liegen.

Das Volumen der im speziellen Fall verwendeten Mikro-Tropfen wird in Abhängigkeit vom Volumen des Kristalls gewählt. Dabei betragen die Volumen der Mikrotropfen weniger als 50 %, z.B. 1 bis 20 %, des Kristallvolumens und vorzugsweise von 1, stärker bevorzugt von 5 bis 10 % des Kristallvolumens.

Die Tropfenerzeugung mittels einer Piezopipette ist nur ein Beispiel für eine Mikrodosiervorrichtung. Es können auch andere Vorrichtungen verwendet werden, die in der Lage sind, Mikro-Tropfen zu erzeugen.

So kann z.B. auch ein Mikrodosiersystem verwendet werden, das eine Kapillare und ein in der Kapillare angeordnetes Mikroventil umfaßt. Dabei wird die Flüssigkeit unter Druck aus einem Vorratsgefäß auf das Mikroventil gepreßt, das von einem Steuergerät elektrisch innerhalb eines kurzen Zeitintervalls geöffnet und danach wieder geschlossen wird, um die Tropfen zu erzeugen. Die Begrenzung der Tropfengröße ergibt sich hier durch die noch steuerbare Öffnungsdauer des Ventils.

Als Mikrodosiersystem kann bei einer anderen Ausführungsform auch ein Zerstäuber dienen. Ein Zerstäuber hat allerdings gegenüber den oben beschriebenen Lösungen den Nachteil, daß das Ausrichten der Tropfen auf den Kristall schwieriger ist. Daher wird vorteilhafter Weise dem Zerstäuber ein Mittel nachgeordnet, das die Orientierung der aus dem Zerstäuber erhaltenen Mikro-Tropfen auf den Kristall sicherstellt.

Es ist gemäß einer weiteren Ausführungsform der erfindungsgemäßen Vorrichtung auch denkbar, daß die Mikrodosier-Vorrichtung aus einem "Loop", bspw. einer Schlaufe, besteht, mit dem einzelne Tropfen (oder nur ein Tropfen) auf den Kristall durch bspw. Abschütteln oder Abtropfenlassen von dem "Loop" aufgebracht werden. Es muß allerdings bei dieser Lösung der erfindungsgemäßen Aufgabe sichergestellt sein, daß die aufgebrachten Tropfenvolumina klein genug für die Proteinkristalle (im Sinn der voranstehend offenbarten Volumenverhältnisse von Kristall zu Tropfen) sind.

Auch alle weiteren technischen Möglichkeiten, Mikro-Tropfen entsprechender Größe zu erzeugen, sind Lösungen im Sinne der vorliegenden Erfindung.

Bei einer Ausführungsform des erfindungsgemäßen Verfahrens wird ein Proteinkristall zunächst an dem freien Auflagenende der Haltekapillare 2 befestigt. Anstelle der Haltekapillare 2 kann auch ein sogenannter "Loop", also eine Art Schlaufe, verwendet werden, in dem der Proteinkristall befestigt ist. Der Proteinkristall ist dabei frei von jeglicher Oberflächenlösung und damit zugänglich für Lösungen, die von außen direkt mittels des Mikrodosiersystems aufgebracht werden können. Durch die Halterung 1 wird nun typischerweise eine Gasatmosphäre um den Proteinkristall 2 herum erzeugt, indem ein Gasstrom definierter Zusammensetzung und Temperatur durch den Gaskanal 6 der Halterung 1 geführt wird. Bei dem beschriebenen Verfahren wird es sich typischerweise um einen Luftstrom, ggf. unter Beimischung anderer gasförmiger Substanzen, mit einem geregelten Feuchtigkeitsgehalt (d.h. Wassergehalt) und einer geregelten Temperatur handeln.

In die Kristallstruktur des Proteinkristalls soll nun ein Inhibitor eingebracht werden, der Bestandteil einer Substanz ist, die der Lösung zugesetzt wurde, die sich in dem Vorratsgefäß befindet, das mit der Piezopipette verbunden ist. Es hat sich durch Experimente gezeigt, daß lokal auf die Oberfläche des Kristalls aufgebrachte Lösungen (wie bspw. DMSO) mit hoher Inhibitor-Konzentration den Kristall in der Regel nicht schädigen. Nun werden durch das Steuergerät 17 elektrische Spannungspulse an die Piezopipette 12 angelegt und Mikro-Tropfen mit der Inhibitor-Lösung auf den Proteinkristall 2 geschleudert. Durch das Aufspritzen einzelner Mikro-Tropfen bleibt der den Proteinkristall umströmende Gasstrom praktisch unbeeinflußt, so daß der Proteinkristall in seiner stabilen definierten Umgebung verbleibt. Die Erhaltung einer stabilen Umgebung ist insbesondere für die relativ instabilen Proteinkristalle, die durch geringe Gitterkräfte zusammengehalten werden, wichtig, damit die Kristalle nicht zerstört werden, bevor sie z.B. einer röntgenkristallographischen Untersuchung unterzogen werden. Die Feuchtigkeit des den Kristall umgebenden Luftstroms kann nun im Zusammenspiel mit der Größe und Frequenz der über die Mikrodosiervorrichtung auf den Proteinkristall aufgebrachten Tropfen so eingestellt werden, daß der Kristall möglichst sein Volumen nur wenig ändert, indem ein Gleichgewicht zwischen Abdampfen von Flüssigkeit vom Kristall und Zuwachs an Flüssigkeit durch Auftropfen von Flüssigkeit mittels der Mikrodosiervorrichtung erreicht wird. Dadurch wird der Kristall nur minimal belastet und es kann ein schonendes Einbringen des/der Liganden über die lokal aufgetragenen Mikrotropfen erreicht werden. Dieser Vorgang der Einstellung der optimalen Luftfeuchtigkeit bzw. der optimalen Auftropffrequenz durch die Mikrodosiervorrichtung kann über ein Regelelement automatisch geregelt werden, daß entsprechende Änderungen der Feuchtigkeit des Luftstroms und/oder der Auftropffrequenz vornimmt, wenn sich das gemessene Volumen des Kristalls ändert.

Während des Kristallbehandlungsprozesses kann der Kristall gemäß einer bevorzugten Ausführungsform der Erfindung auch mit gepulstem Licht bestrahlt werden, z.B. über ein Stroboskop, um mittels des Videosystems in regelmäßigen Abständen eine Vermessung des Volumens des Tropfens durchführen zu können.

Gemäß einer weiteren Ausführungsform der Erfindung ist es auch denkbar, daß der Kristall, der sich in dem Gasstrom definierter Zusammensetzung befindet, von einer Lösung umgeben ist, so daß die durch die Mikrodosiervorrichtung aufgebrachten Tropfen nicht direkt auf den Kristall, sondern in die den Kristall umgebende Lösung gegeben werden.

Die erfindungsgemäße Vorrichtung bzw. das erfindungsgemäße Verfahren erweisen sich auch dann besonders vorteilhaft, wenn Liganden, bspw. Inhibitoren oder andere Stoffe, in einen Kristall eingebracht werden sollen, die selbst in einer wäßrigen Lösung nur schwer zu lösen sind. Tatsächlich erweist sich eine Reihe von Liganden als in wäßrigen Systemen ausgesprochen schwer löslich, so daß mit dem in der Beschreibungseinleitung beschriebenen klassischen "Soaking"-Verfahren diese Liganden/Inhibitoren nicht in den Kristall eingebracht werden können, da die Konzentration der Liganden/Inhibitoren in der wäßrigen Lösung zu gering ist. Wird nun mittels des Mikrodosiersystems eine wäßrige Lösung, in der diese Liganden und/oder Inhibitoren gelöst sind, auf den Kristall aufgetropft, so verdunstet das Wasser nach jedem Auftropfen vollständig, während der Ligand auf bzw. im Kristall verbleibt. Durch wiederholte Auftropfzyklen können so größere Mengen des (schwer löslichen) Liganden auf den Kristall aufgebracht werden. Der Ligand akkumuliert sich so allmählich auf bzw. im Kristall, bis eine ausreichende Menge des Liganden in den Kristall eingebracht ist und eine zufriedenstellende Ligand-Protein-Komplexbildung (also die Besetzung des Kristalls an den Bindungsstellen der kristallisierten Proteine ausreichend ist, eine Elektronendichte für den Liganden zu bestimmen) erreicht ist.

Der Vorteil bei diesem Verfahren liegt auch darin, daß die Proteinkristalle nicht mit einem weiteren Lösungsmittel versetzt werden müssen und so die Behandlung der empfindlichen Kristalle schonender wird. Außerdem besteht derart nicht die Gefahr, daß der Ligand aufgrund seiner geringen Löslichkeit auf dem Kristall bzw. in den Lösungsmittelkanälen präzipitiert. Bei diesem Verfahren kann die Menge an durch das Mikrodosiersystem aufzutropfender Lösung durch die Konzentration der Lösung sowie einer Abschätzung der Molarität des Proteins im Kristall berechnet werden. Ein weiterer Vorteil des Verfahrens besteht darin, daß man mit Wasser als dem einzigen Lösungsmittel für den Liganden im Vergleich zu anderen Lösungsmitteln oder Flüssigkeiten besonders kleine Tropfengrößen erzielen kann, was insbesondere bei kleinen Proteinkristallen wichtig ist, da erfindungsgemäß die Tropfengröße kleiner als die Größe des Kristalls sein sollte.

Die erfindungsgemäße Vorrichtung zum Behandeln eines Kristalls mit einem Substrat kann auch in eine Röntgenbestrahlungsanlage oder Synchrotronbestrahlungsanlage integriert sein, so daß es möglich wird, während der Behandlung des Proteinkristalls mit der Substanz Beugungsbilder des Kristalls aufzunehmen und so den Behandlungsprozeß, d.h. die sukzessive Besetzung der Bindungsstellen des Kristalls, "online" zu beobachten. Hierzu kann die Halterung 1 z.B. an einem Goniometer einer Röntgen- oder Synchrotron-Bestrahlungsanlage befestigt werden. Der Proteinkristall kann vor der röntgenkristallographischen Untersuchung auch eingefroren werden, was in der Regel unter Verwendung von flüssigem Stickstoff erfolgt (sogenannte Cryo-Kristallographie). Hierdurch werden bei röntgenkristallographischen Untersuchungen die Intensitäten der Reflexe des Beugungsbildes ermittelt und schließlich unter Verwendung der Phaseninformation, z.B. aus isomorpher Ersetzung oder MAD ("multiple anomalous scattering"), die Elektronendichte der Struktur ermittelt werden.

Selbstverständlich können auch andere physikalische, insbesondere spektroskopische, Messungen mit Hilfe der erfindungsgemäßen Vorrichtung an dem Kristall durchgeführt werden. So kann die erfindungsgemäße Vorrichtung z.B. auch mit einer Anlage zur Aufnahme einer Absorptionsspektrums kombiniert werden, um das Absorptionsspektrum des Kristalls aufzunehmen.

Gemäß einer weiteren bevorzugten Ausführungsform der Erfindung kann auch oder nur dem durch die Halterung 1 geführten Gasstrom ein Lösungsvermittler beigefügt werden, der sich für die in den Proteinkristall einzubringende Substanz eignet, also z.B. ein Lösungsvermittler für einen schwerlöslichen Liganden. Hierzu kann zusätzlich ein Verdampfer vorgesehen sein, um den Lösungsvermittler vor der Einleitung in den Gaskanal 9 der Halterung 1 zu verdampfen. Auch kann eine Vorrichtung vorgesehen sein, die dazu dient, die Konzentration des Lösungsvermittlers im Gasstrom variabel einzustellen und den erforderlichen Bedingungen anzupassen. So läßt sich eine im Gegensatz zum klassischen "Soaking"-Prozeß sehr schonende Zufuhr von Lösungsvermittler zu dem Proteinkristall erreichen. Während der Zufuhr des den Lösungsvermittler enthaltenden Gasstroms kann dann über die Piezopipette die Ligandenlösung auf den Proteinkristall in Mikro-Tropfenform aufgebracht werden. Insgesamt ergibt sich also erfindungsgemäß die Möglichkeit nur der als Mikrotropfen aufzubringenden Ligandenlösung Lösungsvermittler oder nur dem zugeführten Gasstrom beizumischen . Ggf. können beide Alternativen auch kombiniert werden, so dass sowohl im Mikrotropfen als auch im Gasstrom der Lösungsmittelvermittler (gleich oder verschieden) zugesetzt werden.

Auch kann die über die Mikro-Tropfen mittels des Mikrodosiersystems aufgebrachte Lösung mehrere verschiedene Substanzen enthalten, mit denen der Kristall behandelt werden soll. Es kann sich dabei zum Beispiel um mehrere Liganden, bspw. um mehrere Substrate oder um ein Substrat und um einen katalytisch wirkenden Liganden, handeln, die in einer Lösung gelöst sind, die mittels einer Piezopipette auf den Kristall aufgebracht werden soll.

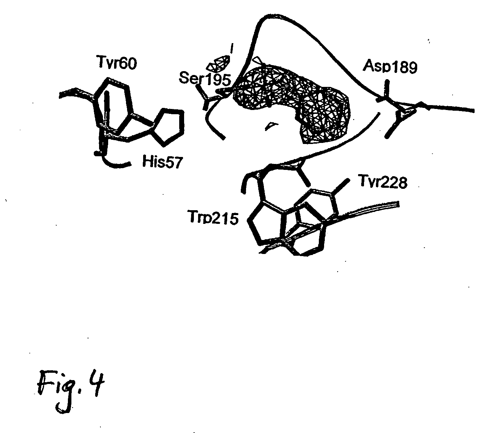

Gemäß einer weiteren Ausführungsform kann die Piezopipette auch mit einem speziellen Flüssigkeitzufuhrsystem versehen sein, mit dem es möglich ist, die Zufuhr verschiedener Flüssigkeiten in die Piezopipette zeitlich in gewünschter Weise zu steuern. In der Fig. 3 ist ein solches Flüssigkeitszufuhrsystem darstellt. Das in der Fig. 3 dargestellte Flüssigkeitszufuhrsystem umfaßt eine Präzisionsspritze 40, die aus einem Zylinder 41 besteht, in dem ein über einen (in der Fig. 3 nicht dargestellten) Motor angetriebener Kolben 42 hin- und herlaufen kann. Wenn der Kolben nach unten läuft, können verschiedene Flüssigkeiten aus den Flüssigkeitsbehältem 43, 44, 45 oder 46 in den Zylinder gesaugt werden, wenn eines der entsprechenden elektrisch ansteuerbaren Ventile 47, 48, 49 bzw. 50 geöffnet wird und zusätzlich das vor dem Zylinder liegende elektrisch steuerbare Ventil 51 geöffnet wird. Wird das Ventil 51 dann wieder geschlossen, das am Auslaß des Zylinders liegende elektrisch steuerbare Ventil 52 geöffnet und der Kolben 42 nach oben getrieben, so kann die angesaugte Flüssigkeit über die zur Piezopipette führende Flüssigkeitszufuhrleitung 53 zur Piezopipette geführt werden, um dann schließlich in Tropfenform auf den Kristall gegeben werden zu können.

Die Behälter 45 und 46 können z.B. zwei verschiedene Lösungen mit verschiedenen Liganden enthalten, die mit dem Protein des zu betropfenden Kristall einen Komplex bilden sollen. Die Behandlung des Kristalls kann dabei z.B. so erfolgen, daß zunächst die Lösung 1 aus dem Behälter 45 und danach die Lösung 2 aus dem Behälter 46 auf den Kristall aufgetropft wird. Zwischen den beiden Lösungen kann eine Reinigungslösung durch die Leitungen gespült werden, die sich in dem Behälter 44 befindet. Der weitere Behälter 47 dient als Abfallbehälter, um Flüssigkeitsmengen aufzunehmen, die nicht mehr benötigt werden und aus dem Zufuhrsystem entfernt werden müssen. Durch geeignete zeitliche Ansteuerung der Ventile 47 - 52 und des Kolbens 42 können nun der Piezopipette die gewünschten Lösungen in der gewünschten Menge zugeführt werden.

Gemäß einer weiteren Ausführungsform der Erfindung können auch mehrere Mikrodosiersysteme, z.B. mehrere Piezopipetten, verwendet werden, mit denen jeweils unterschiedliche oder auch identische Substanzen (bspw. an zwei verschiedenen, lokal abgegrenzten Bereichen des Kristalls) auf den Kristall aufgebracht werden. Eine solche Anordnung kann z.B. von Vorteil sein, wenn zwei verschiedene Liganden in eine Proteinkristallstruktur eingebracht werden sollen. Die Liganden werden dann in verschiedenen Lösungen gelöst, die in die beiden Flüssigkeitsvorratsbehälter zweier Piezopipetten gegeben werden. Über die beiden Piezopipetten werden dann die beiden Lösungen mit den verschiedenen Liganden in Mikro-Tropfenform auf den Proteinkristall aufgebracht. Dabei können über das mit einer Piezopipette jeweils verbundene Steuergerät, das die Tropfenerzeugung steuert, unterschiedliche Spannungsimpulse und Spannungspulsfolgen an die Piezopipetten angelegt werden, um so eine optimale Form und Frequenz der Mikro-Tropfen zu erreichen, die für den jeweiligen Liganden ideal ist.

Die Verwendung von zwei Mikrodosiersystemen, mit denen getrennt zwei verschiedene Substanzen aufgebracht werden, die erst auf dem Kristall zusammentreffen, ist insbesondere auch dann vorteilhaft, wenn das kristallisierte Protein Katalysatorfunktion für die beiden Substanzen, die beide als Reaktanden im kristallisierten Protein gebunden werden, hat. Erfolgt das Aufspritzen der beiden Reaktanden separat durch zwei Mikrodosiersysteme während der Röntgenbestrahlung des Proteinkristalls, kann die Reaktion der Reaktanden unter katalytischem Einsatz der kristallisierten Proteine verfolgt werden. Eine Voraussetzung für eine derartige röntgenkristallographische Untersuchung ist natürlich die Stabilität des Kristalls, d.h., daß der Kristall seine Struktur nicht durch strukturelle Umlagerungen der kristallisierten Proteine verlieren darf, da er dadurch auch sein Diffraktionsvermögen verlieren würde.

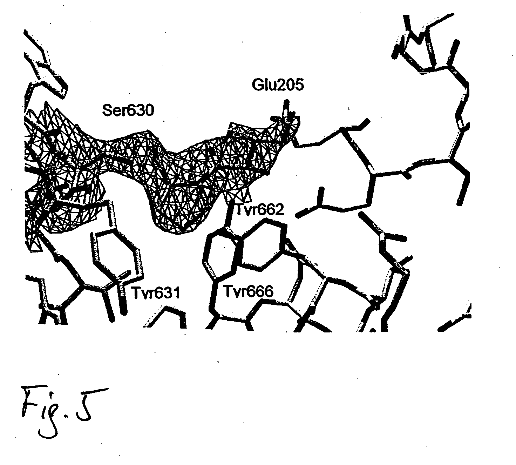

Die vorliegende Erfindung wird durch die Figuren 4 und 5 näher erläutert:

### Figur 4:

Der kovalent gebundene Inhibitor sowie einzelne Aminosäuren in der Umgebung des Aktiven Zentrums des Thrombins um Ser195 sind als stick-Modell dargestellt. Sauerstoffatome sind rot, Schwefelatome gelb, Stickstoffatome blau und Kohlenstoffatome grau dargestellt. Der Inhibitor ist zusätzlich von seiner 2Fₒ-F_{c}-Elektronendichte (kontouriert bei 1σ) überlagert. Der Inhibitor ist in seiner Elektronendichte eindeutig definiert.

In der experimentell bestimmten Elektronendichte ist deutlich die kovalente Bindung des PMSFs am Ser195 zu erkennen (Figur 4), die sich von der des ursprünglich im Kristall gebundenen Benzamidins signifikant unterscheidet. Damit wurde der Beweis geführt, daß der Ansatz des Auftropfens von Picoliter-Tropfen auf einen Proteinkristall unter Verwendung des Free Mounting Systems funktioniert

### Figur 5:

Das Spaltprodukt Pro-Ile des Inhibitors Diprotin A sowie einzelne Aminosäuren in der Umgebung des Aktiven Zentrums der DPIV um Ser630 sind als stick-Modell dargestellt. Sauerstoffatome sind rot, Stickstoffatome blau und Kohlenstoffatome grau dargestellt. Der Inhibitor sowie Ser630, das kovalent an den Inhibitor gekoppelt ist, ist zusätzlich von seiner 2Fₒ-F_{c}-Elektronendichte (kontouriert bei 1σ) überlagert. Der Inhibitor ist in seiner Elektronendichte eindeutig definiert (Figur 5).

Vom eingesetzten Tripeptid mit der Sequenz Ile-Pro-Ile ist das C-terminale Isoleucin abgespalten, während das Dipeptid kovalent mit Ser630 weiterhin verknüpft ist und nicht abgespalten wird. Insofern verhält sich Diprotin A eher wie ein Suizid-Substrat und nicht wie ein Inhibitor.

Die vorliegende Erfindung wird durch die nachfolgenden Ausführungsbeispiele näher beschrieben.

### Ausführungsbeispiele

### 1. Ausführungsbeispiel

### Komplex mit einer Fragmentspezies, Trägerflüssigkeit: Wasser

Es wurde Benzamidin als Ligand und Faktor Xa als Zielenzym eingesetzt. Benzamidin wurde in Wasser in einer 100 mM Konzentration gelöst. Der Faktor-Xa-Kristall wurde in der FMS-Apparatur bei der zuvor ermittelten relativen Feuchte des Muttertropfens von 95% frei montiert. Durch zueinander orthogonale Projektionsaufnahmen konnte das Volumen des Kristalls montiert werden, typische Volumina von Proteinkristallen betragen 1 nl (100 µm x 100 µm x 100 µm). Die Konzentration des Proteins im Kristall betrug ca. 1000 mg/ml, entsprechend einer Konzentration von ca. 30 mM.

Mittels stroboskopischer Aufnahmen wurde der Tropfendurchmesser und damit das Tropfenvolumen bestimmt und zu 5 pl eingestellt. Demgemäß war ein gesamtes Auftropfvolumen von 333 pl entsprechend 67 Tropfen nötig, um alle primären Bindestellen im Kristall äquimolar zu besetzen. Faktor Xa weist eine sekundäre Benzamidin-Bindestelle schwacher Affinität auf, so dass zur (einfachen) Besetzung beider Bindestellen ein theoretisches Aufspritzvolumen von 666 pl nötig war. Zur vollständigen Besetzung der sekundären Bindestelle wurde ein zehnfacher Ligandenüberschuß eingestellt, weswegen insgesamt 1340 Tropfen auf den Kristall aufgespritzt wurden. Die resultierende Ligandkonzentration im Kristall betrug somit 600 mM.

Während des Aufspritzvorgangs wurde die Projektion des Kristallvolumens verfolgt. Der Aufspritzvorgang wurde ausgesetzt, wenn das Kristallvolumen um mehr als 10% vom Ausgangsvolumen zunahm, und setzte erst wieder ein, wenn das Kristallvolumen sich bis auf 2% dem Ausgangsvolumen angenähert hatte. Nach Beendigung des Aufspritzvorgangs wurde der Kristall vollständig an den Ausgangsfeuchte-Gleichgewichtszustand äquilibriert, was durch die asymptotische Angleichung des Kristallvolumens an das Ausgangsvolumen verfolgt wurde. Der Kristall wurde sodann durch Schockgefrieren in flüssigem Stickstoff stabilisiert und röntgenkristallographisch vermessen.

Zur Berechnung des aufzubringenden Flüssigkeitsvolumens lässt sich allgemein feststellen, dass die Berechnung des aufzubringenden Flüssigkeitsvolumens, wie folgt, erfolgt: Die insgesamt aufzutropfende Flüssigkeitsmenge hängt von (i) der Konzentration des Liganden in der Trägerflüssigkeit; (ii) dem Kristallvolumen und (iii) der Konzentration der Proteinbindestellen ("aktive Zentren") ab. Um schließlich die benötigte Anzahl von aufzuspritzenden Piko-Tropfen zu ermitteln muß (iv) das Tropfenvolumen bestimmt werden. Dabei geht die weitergehende Annahme ein, dass die effektiv-wirksame Ligandenmenge der aufgespritzten Ligandenmenge entspricht, insbesondere also keine Präzipitation des Liganden an der Kristalloberfläche auftritt. Ein solches Präzipitationsrisiko besteht bei schlecht wasserlöslichen Substanzen. Diese Substanzen werden oftmals unter Verwendung von Lösungsmitteln (z.B. DMSO) auf den Kristall aufgespritzt, wobei die Ligandenkonzentration so eingestellt wird, dass keine Präzipitation an der Kristalloberfläche auftritt. Die korrekte Konzentrationseinstellung und das daraus resultierende präzipitationsfreie Aufspritzen werden unter einem Mikroskop kontrolliert.

Bestimmung der experimentellen Parameter: (i) Die Konzentration wird definiert eingestellt, z.B. durch Einwaage; (ii) Das Kristallvolumen wird durch eine Serie von Projektionsaufnahmen bei verschiedenen Kristallorientierungen durch das Verfahren der Rückprojektion experimentell bestimmt. (iii) Proteinkristalle enthalten einen Wassergehalt von typischerweise 50%. Dies entspricht einer Proteinkonzentration von ca. 1000 mg/ml. Unter Verwendung des Molekulargewichts des Proteins und Berücksichtigung der Anzahl der (aktiven) Proteinbindestellen kann die Konzentration der Bindestellen berechnet werden. Beispielsweise wäre diese Konzentration bei einem 100 kDa Protein mit einer active site 10 mM. (iv) Unter Verwendung eines Stroboskops kann die Tropfenprojektion vermessen werden. Da der Tropfen in sehr guter Näherung kugelförmig ist, kann das Tropfenvolumen einfach berechnet werden.

Zu beachten ist, dass für die Berechnung der benötigten Tropfenanzahl bei (ü) und (iv) nicht die absoluten sondern nur die relativen Volumina ausschlaggebend sind.

### 2. Ausführungsbeispiel

### Komplex mit einer Fragmentspezies, Trägerflüssigkeit: DMSO

Es wurde PMSF (Phenylmethylsulfonylfluorid) als Ligand und Faktor Xa als Zielenzym eingesetzt. Bei Verwendung von DMSO muß insbesondere bei schlechten löslichen Verbindungen darauf geachtet werden, dass die mit DMSO gelöste Substanz nach dem Aufspritzen auf den Kristall nicht präzipitiert. Die Löslichkeit typischer chemischer Substanzen hängt vom DMSO-Anteil parabolisch ab. Dieser nicht-lineare Zusammenhang begründet die Präzipitationsgefahr der Substanz auf dem Kristall bei Vermischung des Löslichkeitsvermittlers DMSO mit dem Kristallwasser. Daher wurde in einem Vorversuch die Löslichkeit der Substanz bei 50% DMSO ermittelt. Dies ist die maximale Konzentration, die in 100% DMSO aufgebracht werden kann. Für PMSF zeigte sich, dass PMSF bei 50% DMSO zu (mindestens) 10 mM gelöst war. Die Substanz wurde daher in 100% DMSO gelöst und mit 10 mM Konzentration beim Aufspritzexperiment eingesetzt. Bei einem Kristallvolumen von 1 nl und einer einzigen Proteinbindestelle mußten daher 30 nl für äquimolare Besetzung aufgebracht werden, für doppelte Besetzung (einfachen Überschuss) sind theoretisch 60 nl nötig. Bei Verwendung von 10 pl Tropfenvolumina wurden 6000 Tropfen auf den Kristall aufgespritzt. Der Vorgang wurde, wie im Anwendungsbeispiel 1 gezeigt, über die Messung der Kristallprojektion verfolgt und kontrolliert.

### 3. Ausführungsbeispiel

### Komplex mit mehreren verschiedenen Fragmentspezies, Trägerflüssigkeit: DMSO

Das Zielenzym war DPIV (Dipeptidylpeptidase IV). Verschiedene Fragmente wurden in einem Cocktail miteinander in Lösung gebracht. In einem Cocktail (i) wurde Moleküle von zwei verschiedenen Molekülspezies miteinander kombiniert, und zwar ein Mimetikum (verändertes Peptidrückgrat (Vinylderivat) des N-terminalen Dipeptids eines Substrats der DPIV (mit freiem N-Terminus) und andererseits ein Mimetikum (ebenfalls ein Dipeptid mit acetyliertem, blockiertem N-Terminus, ebenfalls als Gerüsmimetikum - Vinylderivat). In einem zweiten Cocktail (ii) wurden jeweils fünf verschiedenen Molekülspezies (die sich in bezug auf die beiden Seitenketten der beiden Dipeptidmimetika unterschieden) der beiden Mimetikaklassen, also insgesamt 10 Molekülspezies, in einem Cocktail kombiniert.

In einem Vorversuch wurde entsprechend Beispiel 2 die (Mindest-)Löslichkeit jeder Substanz bei 50% DMSO ermittelt, die hier zu 10 mM bestimmt wurde. Die DPIV kristallisiert als Tetramer mit einem Molekulargewicht von ca. 400 kDa, mit vier Bindestellen pro Tetramer. Die Konzentration der DPIV-Bindungsstellen im Kristall ist daher 10 mM. Bei einem Kristallvolumen von 1 nl wurde daher insgesamt 1 nl einer 100% DMSO Cocktail-Mischung aufgetragen, die jede Substanz mit einer 10 mM Konzentration gelöst hatte. Das weitere Vorgehen entsprach dann dem der Beispiele 1 und 2.

Auf erfindungsgemäße Weise lassen sich damit Fragmente an den Kristall binden, die mit klassischen "Soaking"-Prozessen nicht gebunden werden können. Zudem ist ein solches erfindungsgemäßes Verfahren im Vergleich zu bisherigen "Soaking"-Prozessen mit geringerem Zeitaufwand verbunden, da wegen der schonenderen Kristallbehandlung weniger Versuche unternommen werden müssen, um die Kristallbehandlung erfolgreich zum Abschluß zu bringen. Insbesondere aber lassen sich mit dem erfindungsgemäßen Verfahren schwach bindende (bspw. 10⁻³ M) Substanzen mit Fragmentcharakter identifizieren, die sich in Lösung nicht mit einem Kristall komplexieren ließen, da Proteine in Lösung mindestens 100fach niedriger konzentriet sind als in einem entsprechenden Proteinkristall. Damit eignet sich das erfindungsgemäße Verfahren zum Fragment-basierten de novo Wirkstoffdesign durch Verknüpfung von verschiedenen erfindungsgemäß aufgefundenen Fragmenten (und damit einer entsprechenden Erhöhung der Bindungsaffinität). Schließlich kann das erfindungsgemäße Verfahren auch zur Fragment-basierten Wirkstoffverfeinerung genutzt werden, indem die aufgefundenen Fragmente oder das aufgefundene Fragment mit einem bekannten Inhibitor bspw. verknüpft oder ein bekannter Inhibitor strukturell modifiziert wird und dadurch die Affinität verbessert werden kann. Damit sind erfindungsgemäße Verfahren auch Bestandteil von allgemeinen Verfahren, die zur Identifikation von Liganden eines Zielproteins dienen können.

## Patentansprüche

1. Verfahren zum Behandeln eines Kristalls mit einer Lösung, enthaltend eine oder mehr Molekülspezies, wobei die Moleküle ein Molekulargewicht von < 500 Da aufweisen, mit den folgenden Schritten:
- es wird der Kristall auf einer Halterungsvorrichtung befestigt und
- es werden Mikrotropfen der Flüssigkeit auf den Kristall aufgebracht.

2. Verfahren zum Behandeln eines Kristalls nach Anspruch 1, wobei die in der Lösung enthaltenden Moleküle ein Molekulargewicht von < 200 Da aufweisen.

3. Verfahren zum Behandeln eines Kristalls nach Anspruch 1 oder 2, wobei die in der Lösung enthaltenden Moleküle ein Molekulargewicht von < 100 Da aufweisen.

4. Verfahren zum Behandeln eines Kristalls nach einem der vorgenannten Ansprüche, wobei der Kristall ein Proteinkristall ist.

5. Verfahren zum Behandeln eines Kristalls nach einem der vorgenannten Ansprüche, wobei die in der Lösung enthaltenen Moleküle als Liganden an die Proteine im Proteinkristall binden, vorzugsweise mit einer Affinität zwischen 10⁻³ und 10⁻⁴ M.

6. Verfahren zum Behandeln eines Kristalls nach einem der vorgenannten Ansprüche, wobei die in der Lösung enthaltenen Moleküle bzw. die Moleküle mindestens einer in der Lösung enthaltenen Molekülspezies mindestens ein elektronenreiches oder anomales Röntgenstreuzentrum, vorzugsweise ein Schwer(metall)atom, aufweisen.

7. Verfahren nach einem der vorgenannten Ansprüche, bei dem darüber hinaus während des Aufbringens der Mikro-Tropfen auf den Kristall um den Kristall herum eine definierte Umgebung erzeugt wird.

8. Verfahren nach Anspruch 7, bei dem das Erzeugen der definierten Umgebung das Erzeugen eines Gasstroms definierter Zusammensetzung um den Kristall herum umfaßt.

9. Verfahren nach Anspruch 8, bei dem der Gasstrom aus einem Luftstrom mit kontrollierter Luftfeuchtigkeit besteht.

10. Verfahren nach Anspruch 8, bei dem der Gasstrom während des Auftropfens geregelt wird.

11. Verfahren nach einem der Ansprüche 9 oder 10, bei dem die Luftfeuchtigkeit des Gasstroms und die Frequenz, mit der die Tropfen durch das Mikrodosiersystem auf den Kristall aufgetropft werden, während des Auftropfens so aufeinander abgestimmt werden, daß der Kristall möglichst wenig belastet wird.

12. Verfahren nach einem der Ansprüche 8 bis 11, bei dem der Gasstrom einen Lösungsvermittler in kontrollierter Konzentration für eine auf den Kristall aufzubringende Substanz umfaßt.

13. Verfahren nach einem der Ansprüche 1 bis 12, bei dem das Volumen der Mikro-Tropfen kleiner als das Volumen des Kristalls ist.

14. Verfahren nach Anspruch 13, bei dem die Mikro-Tropfen der Lösung ein Volumen zwischen 1 nl und 100 pl, vorzugsweise zwischen 100 pl und 20 pl und noch bevorzugt zwischen 20 pl und 4 pl aufweisen.

15. Verfahren nach einem der vorgenannten Ansprüche 1 bis 14, wobei die die Molekülspezies enthaltende und auf den Kristall aufgebrachte Lösung eine wässrige Lösung oder eine zumindest teilweise organische Lösungsmittel umfassende Lösung ist.

16. Verfahren nach Anspruch 15, wobei die die Molekülspezies enthaltende Lösung aus einem flüchtigen organischen Lösungsmittel besteht oder dieses enthält.

17. Verfahren nach Anspruch 16, wobei das Lösungsmittel DMSO enthält oder aus DMSO besteht.

18. Verfahren nach Anspruch 15 oder 16, wobei das die Molekülspezies enthaltende Lösungsmittel ein bei einer Temperatur von weniger als 100°C siedendes, bevorzugt vollständig flüchtiges, organisches Lösungsmittel ist oder dieses enthält.

19. Verfahren nach einem der Ansprüche 15 bis 18, wobei das Lösungsmittel DMSO, Trifluorethanol, Aceton, Chloroform und/oder Methanol enthält.

20. Verfahren nach einem der vorgenannten Ansprüche, wobei die in der auf den Kristall aufzubringenden Lösung enthaltenen Moleküle schwer wasserlöslich sind.

21. Verfahren nach einem der vorgenannten Ansprüche, wobei die Lösung einen Cocktail von mindestens 3, stärker bevorzugt mindestens 10, noch stärker bevorzugt mindestens 20 und am stärksten bevorzugt mindestens 50 verschiedenen Molekülspezies enthält.

22. Verfahren nach einem der vorgenannten Ansprüche, wobei die Lösung die mindestens eine Molekülspezies in einer Konzentration von 10⁻¹ bis 10⁻³M enthält.

23. Verfahren nach einem der vorgenannten Ansprüche, wobei dem Verfahren ein Verfahrensschritt vorgeschaltet ist, mit dem potentiell an eine Zielstruktur bindende Fragmente identifiziert werden, insbesondere ein Verfahrensschritt, der auf einem spektroskopischen Verfahren, bspw. NMR-Spektroskopie oder Oberflächenplasmonresonanzspektroskopie, oder einem in silico Docking Verfahren beruht.

24. Verfahren nach einem der Ansprüche 1 bis 23, bei dem der Gasstrom eine oder mehrere Substanzen enthält, die einen oder mehrere Liganden und/oder Inhibitoren enthält bzw. enthalten.

25. Verfahren zur Ermittlung einer kristallographischen Struktur eines Komplexes eines bspw. Proteins und mindestens einer Molekülspezies, wobei (a) die Verfahrensschritte nach einem der Ansprüche 1 bis 24 durchgeführt werden, (b) der Kristall mit Röntgenstrahlung oder Synchrotronstrahlung bestrahlt wird und (c) das Beugungsbild des Kristalls aufgenommen wird.

26. Verfahren zur Ermittlung einer kristallographischen Struktur nach Anspruch 25, wobei (d) unter Verwendung von Phaseninformation und der Intensitäten der Reflexe im Beugungsbild eine Elektronendichtekarte errechnet und die Bindungsstelle und Positionierung der mindestens einen gebundenen Molekülspezies in der bspw. Proteinstruktur bestimmt wird.

27. Verfahren nach Anspruch 26, bei dem die Phaseninformation durch die Verwendung von Schwermetallatomderivaten ("isomorphe Ersetzung"), "molecular replacement" oder MAD (multiple anomalous scattering) erhalten wird.

28. Verfahren zur Ermittlung einer kristallographischen Struktur nach Anspruch 26 oder 27, wobei die Bindungsstelle und Positionierung der mindestens einen gebundenen Molekülspezies in der bspw. Proteinstruktur aus der Differenz der Elektronendichten von nicht-komplexierter und komplexierter Struktur durch eine Differenzelektronendichtekarte bestimmt wird.

29. Verfahren nach einem der Ansprüche 25 bis 27, bei dem die Bestrahlung mit monochromatischer Röntgenstrahlung oder mit Synchrotronstrahlung während der Behandlung des Kristalls mit der Lösung stattfindet.

30. Verfahren zur Identifikation von ein kristallisiertes Protein bindenden Molekülen, bei dem (a) mindestens eine Molekülspezies nach einem Verfahren nach einem der Ansprüche 1 bis 24 auf den Kristall aufgebracht wird, (b) in einem zeitlichen Abstand von variabler Länge Beugungsintensitäten gemessen werden und (c) diese im zeitlichen Abstand gemessenen Beugungsintensitäten entsprechend ihrer zeitlichen Abfolge miteinander verglichen werden.

31. Verfahren zur Identifikation von einem eine Zielstruktur bindenden Liganden, wobei (a) ein Verfahren nach einem der Ansprüche 1 bis 24 durchgeführt wird, (b) nach einem Verfahren nach einem der Ansprüche 25 bis 29 die Struktur von mindestens einem Komplexes mit mindestens zwei Fragmenten ermittelt wird, (c) Linker zwischen den mindestens zwei Fragmenten zu einem Liganden ermittelt wird/werden und (d) ein Ligand, enthaltend die mindestens zwei Fragmente und den mindestens einen Linker, synthetisiert wird.

32. Verfahren nach einem der Ansprüche 1 bis 24, wobei das Verfahren unter Verwendung einer Vorrichtung zum Behandeln eines Kristalls mit einer Flüssigkeit mit einer Halterung zur Befestigung des Kristalls und mindestens einem Mikrodosiersystem, das im Verhältnis zur Halterung so angeordnet ist, daß damit Mikro-Tropfen der Flüssigkeit auf den in der Halterung befestigten Kristall aufgebracht werden können, durchgeführt wird.

33. Verfahren nach Anspruch 32, wobei die verfahrensgemäß eingesetzte Vorrichtung darüber hinaus ein Mittel umfaßt, mit dem während des Auftropfens der Flüssigkeit um den Kristall herum eine definierte Umgebung erzeugt werden kann.

34. Verfahren nach einem der Ansprüche 32 oder 33, bei dem das Mittel das Erzeugen der definierten Umgebung dadurch erlaubt, dass ein Gasstrom definierter Zusammensetzung um den Kristall herum erzeugt wird.

35. Verfahren nach Anspruch 32 oder 34, bei der die Halterung darüber hinaus so ausgebildet ist, daß durch die Halterung der Gasstrom so geführt werden kann, daß er auf den in der Halterung befestigten Kristall gerichtet ist.

36. Verfahren nach einem der vorhergehenden Ansprüche, bei dem eine Vorrichtung mit einer Halterung aus einem Trägerblock für eine Haltekapillare eingesetzt wird, die ein freies Auflageende für den Kristall besitzt.

37. Verfahren nach Anspruch 36, bei dem eine Vorrichtung mit einer Haltekapillare aus einer Mikropipette eingesetzt wird, in der sich ein Unterdruck erzeugen läßt, um den Kristall zu halten.

38. Verfahren nach einem der Ansprüche 36 oder 37, bei dem der Trägerblock der Halterung der verfahrensgemäß eingesetzten Vorrichtung einen integralen Gaskanal mit einem Mündungsende enthält, das auf das Auflageende der Haltekapillare gerichtet ist.

39. Verfahren nach einem der Ansprüche 34 bis 38, bei dem eine Vorrichtung eingesetzt wird, die darüber hinaus ein Gasmischmittel aufweist, mit dem die Zusammensetzung des Gasstroms variabel eingestellt werden kann.

40. Verfahren nach Anspruch 39, bei dem eine Vorrichtung eingesetzt wird, bei der das Gas aus Luft mit einem bestimmten Feuchtigkeitsgehalt besteht und das Gasmischmittel so ausgebildet ist, daß damit die Luftfeuchtigkeit eingestellt werden kann.

41. Verfahren nach einem der Ansprüche 34 bis 40, bei dem eine Vorrichtung eingesetzt wird, die darüber hinaus ein Lösungsvermittlerbeigabemittel umfaßt, mit dem dem Gasstrom ein Lösungsvermittler für eine in die Kristallstruktur des Kristalls einzubringende Substanz beigemischt werden kann.

42. Verfahren nach Anspruch 41, bei dem eine Vorrichtung eingesetzt wird, die darüber hinaus ein Konzentrationseinstellmittel zur Einstellung der Konzentration des Lösungsvermittlers umfaßt.

43. Verfahren nach einem der Ansprüche 34 bis 42, bei dem eine Vorrichtung eingesetzt wird, die darüber hinaus ein Temperatureinstellmittel umfaßt, mit dem die Temperatur des Gasstroms variabel eingestellt werden kann.

44. Verfahren nach einem der vorhergehenden Ansprüche, bei dem eine Vorrichtung eingesetzt wird, bei der das Mikrodosiersystem so ausgebildet ist, daß es Mikro-Tropfen der auf den Kristall aufzubringenden Flüssigkeit erzeugen kann, die ein Volumen aufweisen, das kleiner als das Volumen des Kristalls ist.

45. Verfahren nach Anspruch 44, bei dem eine Vorrichtung eingesetzt wird, bei der das Mikrodosiersystem so ausgebildet ist, daß es Mikro-Tropfen erzeugen kann, deren Volumen zwischen 10 und 20 Prozent des Kristallvolumens und vorzugsweise zwischen 5 und 10 Prozent des Kristallvolumens beträgt.

46. Verfahren nach einem der Ansprüche 42 oder 43, bei der das Mikrodosiersystem so ausgebildet ist, daß es Mikro-Tropfen erzeugen kann, deren Volumen zwischen 1 nl und 100 pl, vorzugsweise zwischen 100 pl und 20 pl und noch bevorzugt zwischen 20 pl und 4 pl liegt.

47. Verfahren nach einem der vorhergehenden Ansprüche, bei dem eine Vorrichtung eingesetzt wird, bei der das Mikrodosiersystem darüber hinaus ein Flüssigkeitszufuhrsystem aufweist, mit dem verschiedene Flüssigkeiten, die auf den Kristall aufgetropft werden sollen, in zeitlich gesteuerter Weise einem Tropfenerzeugungsteil des Mikrodosiersystems zugeführt werden können.

48. Verfahren nach Anspruch 47, bei dem eine Vorrichtung eingesetzt wird, bei der das Flüssigkeitszufuhrsystem des Mikrodosiersystems umfaßt eine elektrisch ansteuerbare Präzisionsspritze und ein Leitungssystem umfaßt, mit dem die Präzisionsspritze über elektrisch steuerbare Ventile mit verschiedenen Flüssigkeitsvorratsbehältern und mit dem Tropfenerzeugungsteil des Mikrodosiersystems verbunden werden kann, um diesem Flüssigkeit für die Tropfenerzeugung zuzuführen.

49. Verfahren nach einem der vorhergehenden Ansprüche, bei dem eine Vorrichtung eingesetzt wird, bei der das Mikrodosiersystem so ausgebildet ist, daß es eine Piezopipette umfaßt, die das Tropfenerzeugungsteil bildet.
